# EUROPEAN PATENT APPLICATION

(11) **EP 4 660 206 A1**
(43) Date of publication of application: **10.12.2025**
(21) Application number: 24802912.6
(22) Date of filing: 06.05.2024
(51) Int. Cl.: C07K 19/00, C07K 14/82, A61K 39/00, A61P 35/00, G01N 33/68, C07K 16/30, C12N 15/62, A61K 47/68

(54) **TCR MOLECULE TARGETING KRAS G12V MUTATION, CELL, AND USES THEREOF**

(30) Priority: 06.05.2023 CN 202310507375
(71) Applicant: Beijing DCTY Biotech Co., Ltd., Beijing 102200 (CN)
(72) Inventor: WEI, Shao, Beijing 102200 (CN); WANG, Ping, Beijing 102200 (CN); LI, Xiaorui, Beijing 102200 (CN); WANG, Kunfu, Beijing 102200 (CN)
(74) Representative: karo IP
(86) International application number: PCT/CN2024/091149
(87) International publication number: WO 2024/230630

(57) **Abstract**

Provided is a binding protein, comprising a binding domain having antigen specificity for an antigen peptide:HLA complex, wherein the binding domain comprises a T cell receptor (TCR) α-chain variable region and a TCR β-chain variable region. Further provided are uses of the binding protein and a pharmaceutical composition comprising the binding protein in the treatment of cancers associated with the antigen peptide.

## Description

### Cross-reference to related applications

This application claims priority to Chinese Patent Application No. 202310507375.6, filed to China National Intellectual Property Administration on May 6, 2023, which is incorporated herein by reference in its entirety.

### Technical field

The present invention relates to TCR molecule and cells expressing the TCR molecule, particularly to TCR molecule that targets to target cells carrying KRAS G12V mutation and immune effector cells expressing the TCR molecule. The present invention also relates to therapeutic uses of the TCR molecule and the cells.

### Background

The RAS gene family (HRAS, KRAS, NRAS) represents the most prevalent proto-oncogenes capable of driving tumorigenesis. Among these, KRAS mutations-predominantly localized at codon 12-occur in approximately 22-30% of tumors, with particularly high incidence in pancreatic cancer (~60-90%), colorectal cancer (~40%), lung cancer (~10% in China, ~30% in Western populations), cholangiocarcinoma (~17-37%), as well as ovarian and endometrial carcinomas (~10-40%, correlated with pathological subtypes, with higher frequency in serous subtypes). Taking pancreatic cancer as an example, in 2021, an estimated 60,430 new diagnoses and 48,220 deaths were projected. Despite therapeutic advances over the past decade, the 5-year survival rate for pancreatic cancer remains approximately 9% due to limited treatment efficacy. Current treatment of unresectable pancreatic carcinoma primarily involves tumor-reductive chemotherapy, which is constrained by acquired resistance, and the success rate of targeted therapies is also limited. Moreover, pancreatic cancer exhibits resistance to immunotherapy, attributable in part to the disease's low mutational burden resulting in a deficiency of neoantigen-reactive tumor-infiltrating lymphocytes. Research on KRAS-G12V-targeting TCR-T cell therapy provides effective therapeutic approaches to address the current lack of treatments for KRAS-mutant tumors.

### Summary of the invention

In one aspect, the present invention provides a binding protein, comprising a binding domain having antigen specificity for an antigen peptide:HLA complex, wherein the binding domain comprises a T cell receptor (TCR) α chain variable region and a TCR β chain variable region, wherein:
the antigen peptide comprises an amino acid sequence of VVGAVGVGK (SEQ ID NO: 91);
the HLA is HLA-A*11:01;
the CDR3 of the α chain variable region comprises the amino acid sequence of SEQ ID NO: 5, 15, 25, 35, 45, 55, 65, 75, or 85, or an amino acid sequence having at least 80% identity, preferably at least 85% identity, more preferably 90% or 95% identity to the amino acid sequence of SEQ ID NO: 5, 15, 25, 35, 45, 55, 65, 75, or 85;
the CDR3 of the β chain variable region comprises the amino acid sequence of SEQ ID NO: 10, 20, 30, 40, 50, 60, 70, 80, or 90, or an amino acid sequence having at least 80% identity, preferably at least 85% identity, more preferably 90% or 95% identity to the amino acid sequence of SEQ ID NO: 10, 20, 30, 40, 50, 60, 70, 80, or 90.

In some embodiments, the antigen peptide is VVGAVGVGK (SEQ ID NO: 91) or VVVGAVGVGK (SEQ ID NO: 92).

In some embodiments, the α chain variable region comprises: 1) CDR1 comprising the amino acid sequence of SEQ ID NO: 3, CDR2 comprising the amino acid sequence of SEQ ID NO: 4, and CDR3 comprising the amino acid sequence of SEQ ID NO: 5, or an amino acid sequence having at least 80% identity, preferably at least 85% identity, more preferably 90% or 95% identity to the amino acid sequence of SEQ ID NO: 5; 2) CDR1 comprising the amino acid sequence of SEQ ID NO: 13, CDR2 comprising the amino acid sequence of SEQ ID NO: 14, and CDR3 comprising the amino acid sequence of SEQ ID NO: 15, or an amino acid sequence having at least 80% identity, preferably at least 85% identity, more preferably 90% or 95% identity to the amino acid sequence of SEQ ID NO: 15; 3) CDR1 comprising the amino acid sequence of SEQ ID NO: 23, CDR2 comprising the amino acid sequence of SEQ ID NO: 24, and CDR3 comprising the amino acid sequence of SEQ ID NO: 25, or an amino acid sequence having at least 80% identity, preferably at least 85% identity, more preferably 90% or 95% identity to the amino acid sequence of SEQ ID NO: 25; 4) CDR1 comprising the amino acid sequence of SEQ ID NO: 33, CDR2 comprising the amino acid sequence of SEQ ID NO: 34, and CDR3 comprising the amino acid sequence of SEQ ID NO: 35, or an amino acid sequence having at least 80% identity, preferably at least 85% identity, more preferably 90% or 95% identity to the amino acid sequence of SEQ ID NO: 35; 5) CDR1 comprising the amino acid sequence of SEQ ID NO: 43, CDR2 comprising the amino acid sequence of SEQ ID NO: 44, and CDR3 comprising the amino acid sequence of SEQ ID NO: 45, or an amino acid sequence having at least 80% identity, preferably at least 85% identity, more preferably 90% or 95% identity to the amino acid sequence of SEQ ID NO: 45; 6) CDR1 comprising the amino acid sequence of SEQ ID NO: 53, CDR2 comprising the amino acid sequence of SEQ ID NO: 54, and CDR3 comprising the amino acid sequence of SEQ ID NO: 55, or an amino acid sequence having at least 80% identity, preferably at least 85% identity, more preferably 90% or 95% identity to the amino acid sequence of SEQ ID NO: 55; 7) CDR1 comprising the amino acid sequence of SEQ ID NO: 63, CDR2 comprising the amino acid sequence of SEQ ID NO: 64, and CDR3 comprising the amino acid sequence of SEQ ID NO: 65, or an amino acid sequence having at least 80% identity, preferably at least 85% identity, more preferably 90% or 95% identity to the amino acid sequence of SEQ ID NO: 65; 8) CDR1 comprising the amino acid sequence of SEQ ID NO: 73, CDR2 comprising the amino acid sequence of SEQ ID NO: 74, and CDR3 comprising the amino acid sequence of SEQ ID NO: 75, or an amino acid sequence having at least 80% identity, preferably at least 85% identity, more preferably 90% or 95% identity to the amino acid sequence of SEQ ID NO: 75; or 9) CDR1 comprising the amino acid sequence of SEQ ID NO: 83, CDR2 comprising the amino acid sequence of SEQ ID NO: 84, and CDR3 comprising the amino acid sequence of SEQ ID NO: 85, or an amino acid sequence having at least 80% identity, preferably at least 85% identity, more preferably 90% or 95% identity to the amino acid sequence of SEQ ID NO: 85, and
the β chain variable region comprises: 1) CDR1 comprising the amino acid sequence of SEQ ID NO: 8, CDR2 comprising the amino acid sequence of SEQ ID NO: 9, and CDR3 comprising the amino acid sequence of SEQ ID NO: 10, or an amino acid sequence having at least 80% identity, preferably at least 85% identity, more preferably 90% or 95% identity to the amino acid sequence of SEQ ID NO: 10; 2) CDR1 comprising the amino acid sequence of SEQ ID NO: 18, CDR2 comprising the amino acid sequence of SEQ ID NO: 19, and CDR3 comprising the amino acid sequence of SEQ ID NO: 20, or an amino acid sequence having at least 80% identity, preferably at least 85% identity, more preferably 90% or 95% identity to the amino acid sequence of SEQ ID NO: 20; 3) CDR1 comprising the amino acid sequence of SEQ ID NO: 28, CDR2 comprising the amino acid sequence of SEQ ID NO: 29, and CDR3 comprising the amino acid sequence of SEQ ID NO: 30, or an amino acid sequence having at least 80% identity, preferably at least 85% identity, more preferably 90% or 95% identity to the amino acid sequence of SEQ ID NO: 30; 4) CDR1 comprising the amino acid sequence of SEQ ID NO: 38, CDR2 comprising the amino acid sequence of SEQ ID NO: 39, and CDR3 comprising the amino acid sequence of SEQ ID NO: 40, or an amino acid sequence having at least 80% identity, preferably at least 85% identity, more preferably 90% or 95% identity to the amino acid sequence of SEQ ID NO: 40; 5) CDR1 comprising the amino acid sequence of SEQ ID NO: 48, CDR2 comprising the amino acid sequence of SEQ ID NO: 49, and CDR3 comprising the amino acid sequence of SEQ ID NO: 50, or an amino acid sequence having at least 80% identity, preferably at least 85% identity, more preferably 90% or 95% identity to the amino acid sequence of SEQ ID NO: 50; 6) CDR1 comprising the amino acid sequence of SEQ ID NO: 58, CDR2 comprising the amino acid sequence of SEQ ID NO: 59, and CDR3 comprising the amino acid sequence of SEQ ID NO: 60, or an amino acid sequence having at least 80% identity, preferably at least 85% identity, more preferably 90% or 95% identity to the amino acid sequence of SEQ ID NO: 60; 7) CDR1 comprising the amino acid sequence of SEQ ID NO: 68, CDR2 comprising the amino acid sequence of SEQ ID NO: 69, and CDR3 comprising the amino acid sequence of SEQ ID NO: 70, or an amino acid sequence having at least 80% identity, preferably at least 85% identity, more preferably 90% or 95% identity to the amino acid sequence of SEQ ID NO: 70; 8) CDR1 comprising the amino acid sequence of SEQ ID NO: 78, CDR2 comprising the amino acid sequence of SEQ ID NO: 79, and CDR3 comprising the amino acid sequence of SEQ ID NO: 80, or an amino acid sequence having at least 80% identity, preferably at least 85% identity, more preferably 90% or 95% identity to the amino acid sequence of SEQ ID NO: 80; or 9) CDR1 comprising the amino acid sequence of SEQ ID NO: 88, CDR2 comprising the amino acid sequence of SEQ ID NO: 89, and CDR3 comprising the amino acid sequence of SEQ ID NO: 90, or an amino acid sequence having at least 80% identity, preferably at least 85% identity, more preferably 90% or 95% identity to the amino acid sequence of SEQ ID NO: 90.

In some embodiments, 1) the α chain variable region comprises CDR1 comprising the amino acid sequence of SEQ ID NO: 3, CDR2 comprising the amino acid sequence of SEQ ID NO: 4, and CDR3 comprising the amino acid sequence of SEQ ID NO: 5, or an amino acid sequence having at least 80% identity, preferably at least 85% identity, more preferably 90% or 95% identity to the amino acid sequence of SEQ ID NO: 5; the β chain variable region comprises CDR1 comprising the amino acid sequence of SEQ ID NO: 8, CDR2 comprising the amino acid sequence of SEQ ID NO: 9, and CDR3 comprising the amino acid sequence of SEQ ID NO: 10, or an amino acid sequence having at least 80% identity, preferably at least 85% identity, more preferably 90% or 95% identity to the amino acid sequence of SEQ ID NO: 10; 2) the α chain variable region comprises CDR1 comprising the amino acid sequence of SEQ ID NO: 13, CDR2 comprising the amino acid sequence of SEQ ID NO: 14, and CDR3 comprising the amino acid sequence of SEQ ID NO: 15, or an amino acid sequence having at least 80% identity, preferably at least 85% identity, more preferably 90% or 95% identity to the amino acid sequence of SEQ ID NO: 15; the β chain variable region comprises CDR1 comprising the amino acid sequence of SEQ ID NO: 18, CDR2 comprising the amino acid sequence of SEQ ID NO: 19, and CDR3 comprising the amino acid sequence of SEQ ID NO: 20, or an amino acid sequence having at least 80% identity, preferably at least 85% identity, more preferably 90% or 95% identity to the amino acid sequence of SEQ ID NO: 20; 3) the α chain variable region comprises CDR1 comprising the amino acid sequence of SEQ ID NO: 23, CDR2 comprising the amino acid sequence of SEQ ID NO: 24, and CDR3 comprising the amino acid sequence of SEQ ID NO: 25, or an amino acid sequence having at least 80% identity, preferably at least 85% identity, more preferably 90% or 95% identity to the amino acid sequence of SEQ ID NO: 25; the β chain variable region comprises CDR1 comprising the amino acid sequence of SEQ ID NO: 28, CDR2 comprising the amino acid sequence of SEQ ID NO: 29, and CDR3 comprising the amino acid sequence of SEQ ID NO: 30, or an amino acid sequence having at least 80% identity, preferably at least 85% identity, more preferably 90% or 95% identity to the amino acid sequence of SEQ ID NO: 30; 4) the α chain variable region comprises CDR1 comprising the amino acid sequence of SEQ ID NO: 33, CDR2 comprising the amino acid sequence of SEQ ID NO: 34, and CDR3 comprising the amino acid sequence of SEQ ID NO: 35, or an amino acid sequence having at least 80% identity, preferably at least 85% identity, more preferably 90% or 95% identity to the amino acid sequence of SEQ ID NO: 35; the β chain variable region comprises CDR1 comprising the amino acid sequence of SEQ ID NO: 38, CDR2 comprising the amino acid sequence of SEQ ID NO: 39, and CDR3 comprising the amino acid sequence of SEQ ID NO: 40, or an amino acid sequence having at least 80% identity, preferably at least 85% identity, more preferably 90% or 95% identity to the amino acid sequence of SEQ ID NO: 40; 5) the α chain variable region comprises CDR1 comprising the amino acid sequence of SEQ ID NO: 43, CDR2 comprising the amino acid sequence of SEQ ID NO: 44, and CDR3 comprising the amino acid sequence of SEQ ID NO: 45, or an amino acid sequence having at least 80% identity, preferably at least 85% identity, more preferably 90% or 95% identity to the amino acid sequence of SEQ ID NO: 45; the β chain variable region comprises CDR1 comprising the amino acid sequence of SEQ ID NO: 48, CDR2 comprising the amino acid sequence of SEQ ID NO: 49, and CDR3 comprising the amino acid sequence of SEQ ID NO: 50, or an amino acid sequence having at least 80% identity, preferably at least 85% identity, more preferably 90% or 95% identity to the amino acid sequence of SEQ ID NO: 50; 6) the α chain variable region comprises CDR1 comprising the amino acid sequence of SEQ ID NO: 53, CDR2 comprising the amino acid sequence of SEQ ID NO: 54, and CDR3 comprising the amino acid sequence of SEQ ID NO: 55, or an amino acid sequence having at least 80% identity, preferably at least 85% identity, more preferably 90% or 95% identity to the amino acid sequence of SEQ ID NO: 55; the β chain variable region comprises CDR1 comprising the amino acid sequence of SEQ ID NO: 58, CDR2 comprising the amino acid sequence of SEQ ID NO: 59, and CDR3 comprising the amino acid sequence of SEQ ID NO: 60, or an amino acid sequence having at least 80% identity, preferably at least 85% identity, more preferably 90% or 95% identity to the amino acid sequence of SEQ ID NO: 60; 7) the α chain variable region comprises CDR1 comprising the amino acid sequence of SEQ ID NO: 63, CDR2 comprising the amino acid sequence of SEQ ID NO: 64, and CDR3 comprising the amino acid sequence of SEQ ID NO: 65, or an amino acid sequence having at least 80% identity, preferably at least 85% identity, more preferably 90% or 95% identity to the amino acid sequence of SEQ ID NO: 65; the β chain variable region comprises CDR1 comprising the amino acid sequence of SEQ ID NO: 68, CDR2 comprising the amino acid sequence of SEQ ID NO: 69, and CDR3 comprising the amino acid sequence of SEQ ID NO: 70, or an amino acid sequence having at least 80% identity, preferably at least 85% identity, more preferably 90% or 95% identity to the amino acid sequence of SEQ ID NO: 70; 8) the α chain variable region comprises CDR1 comprising the amino acid sequence of SEQ ID NO: 73, CDR2 comprising the amino acid sequence of SEQ ID NO: 74, and CDR3 comprising the amino acid sequence of SEQ ID NO: 75, or an amino acid sequence having at least 80% identity, preferably at least 85% identity, more preferably 90% or 95% identity to the amino acid sequence of SEQ ID NO: 75; the β chain variable region comprises CDR1 comprising the amino acid sequence of SEQ ID NO: 78, CDR2 comprising the amino acid sequence of SEQ ID NO: 79, and CDR3 comprising the amino acid sequence of SEQ ID NO: 80, or an amino acid sequence having at least 80% identity, preferably at least 85% identity, more preferably 90% or 95% identity to the amino acid sequence of SEQ ID NO: 80; or 9) the α chain variable region comprises CDR1 comprising the amino acid sequence of SEQ ID NO: 83, CDR2 comprising the amino acid sequence of SEQ ID NO: 84, and CDR3 comprising the amino acid sequence of SEQ ID NO: 85, or an amino acid sequence having at least 80% identity, preferably at least 85% identity, more preferably 90% or 95% identity to the amino acid sequence of SEQ ID NO: 85; the β chain variable region comprises CDR1 comprising the amino acid sequence of SEQ ID NO: 88, CDR2 comprising the amino acid sequence of SEQ ID NO: 89, and CDR3 comprising the amino acid sequence of SEQ ID NO: 90, or an amino acid sequence having at least 80% identity, preferably at least 85% identity, more preferably 90% or 95% identity to the amino acid sequence of SEQ ID NO: 90.

In another aspect, the present invention provides a binding protein comprising a binding domain, wherein the binding domain comprises a T cell receptor (TCR) α chain variable region and a TCR β chain variable region, wherein: 1) the α chain variable region comprises CDR1 comprising the amino acid sequence of SEQ ID NO: 3, CDR2 comprising the amino acid sequence of SEQ ID NO: 4, and CDR3 comprising the amino acid sequence of SEQ ID NO: 5, or an amino acid sequence having at least 80% identity, preferably at least 85% identity, more preferably 90% or 95% identity to the amino acid sequence of SEQ ID NO: 5; the β chain variable region comprises CDR1 comprising the amino acid sequence of SEQ ID NO: 8, CDR2 comprising the amino acid sequence of SEQ ID NO: 9, and CDR3 comprising the amino acid sequence of SEQ ID NO: 10, or an amino acid sequence having at least 80% identity, preferably at least 85% identity, more preferably 90% or 95% identity to the amino acid sequence of SEQ ID NO: 10; 2) the α chain variable region comprises CDR1 comprising the amino acid sequence of SEQ ID NO: 13, CDR2 comprising the amino acid sequence of SEQ ID NO: 14, and CDR3 comprising the amino acid sequence of SEQ ID NO: 15, or an amino acid sequence having at least 80% identity, preferably at least 85% identity, more preferably 90% or 95% identity to the amino acid sequence of SEQ ID NO: 15; the β chain variable region comprises CDR1 comprising the amino acid sequence of SEQ ID NO: 18, CDR2 comprising the amino acid sequence of SEQ ID NO: 19, and CDR3 comprising the amino acid sequence of SEQ ID NO: 20, or an amino acid sequence having at least 80% identity, preferably at least 85% identity, more preferably 90% or 95% identity to the amino acid sequence of SEQ ID NO: 20; 3) the α chain variable region comprises CDR1 comprising the amino acid sequence of SEQ ID NO: 23, CDR2 comprising the amino acid sequence of SEQ ID NO: 24, and CDR3 comprising the amino acid sequence of SEQ ID NO: 25, or an amino acid sequence having at least 80% identity, preferably at least 85% identity, more preferably 90% or 95% identity to the amino acid sequence of SEQ ID NO: 25; the β chain variable region comprises CDR1 comprising the amino acid sequence of SEQ ID NO: 28, CDR2 comprising the amino acid sequence of SEQ ID NO: 29, and CDR3 comprising the amino acid sequence of SEQ ID NO: 30, or an amino acid sequence having at least 80% identity, preferably at least 85% identity, more preferably 90% or 95% identity to the amino acid sequence of SEQ ID NO: 30; 4) the α chain variable region comprises CDR1 comprising the amino acid sequence of SEQ ID NO: 33, CDR2 comprising the amino acid sequence of SEQ ID NO: 34, and CDR3 comprising the amino acid sequence of SEQ ID NO: 35, or an amino acid sequence having at least 80% identity, preferably at least 85% identity, more preferably 90% or 95% identity to the amino acid sequence of SEQ ID NO: 35; the β chain variable region comprises CDR1 comprising the amino acid sequence of SEQ ID NO: 38, CDR2 comprising the amino acid sequence of SEQ ID NO: 39, and CDR3 comprising the amino acid sequence of SEQ ID NO: 40, or an amino acid sequence having at least 80% identity, preferably at least 85% identity, more preferably 90% or 95% identity to the amino acid sequence of SEQ ID NO: 40; 5) the α chain variable region comprises CDR1 comprising the amino acid sequence of SEQ ID NO: 43, CDR2 comprising the amino acid sequence of SEQ ID NO: 44, and CDR3 comprising the amino acid sequence of SEQ ID NO: 45, or an amino acid sequence having at least 80% identity, preferably at least 85% identity, more preferably 90% or 95% identity to the amino acid sequence of SEQ ID NO: 45; the β chain variable region comprises CDR1 comprising the amino acid sequence of SEQ ID NO: 48, CDR2 comprising the amino acid sequence of SEQ ID NO: 49, and CDR3 comprising the amino acid sequence of SEQ ID NO: 50, or an amino acid sequence having at least 80% identity, preferably at least 85% identity, more preferably 90% or 95% identity to the amino acid sequence of SEQ ID NO: 50; 6) the α chain variable region comprises CDR1 comprising the amino acid sequence of SEQ ID NO: 53, CDR2 comprising the amino acid sequence of SEQ ID NO: 54, and CDR3 comprising the amino acid sequence of SEQ ID NO: 55, or an amino acid sequence having at least 80% identity, preferably at least 85% identity, more preferably 90% or 95% identity to the amino acid sequence of SEQ ID NO: 55; the β chain variable region comprises CDR1 comprising the amino acid sequence of SEQ ID NO: 58, CDR2 comprising the amino acid sequence of SEQ ID NO: 59, and CDR3 comprising the amino acid sequence of SEQ ID NO: 60, or an amino acid sequence having at least 80% identity, preferably at least 85% identity, more preferably 90% or 95% identity to the amino acid sequence of SEQ ID NO: 60; 7) the α chain variable region comprises CDR1 comprising the amino acid sequence of SEQ ID NO: 63, CDR2 comprising the amino acid sequence of SEQ ID NO: 64, and CDR3 comprising the amino acid sequence of SEQ ID NO: 65, or an amino acid sequence having at least 80% identity, preferably at least 85% identity, more preferably 90% or 95% identity to the amino acid sequence of SEQ ID NO: 65; the β chain variable region comprises CDR1 comprising the amino acid sequence of SEQ ID NO: 68, CDR2 comprising the amino acid sequence of SEQ ID NO: 69, and CDR3 comprising the amino acid sequence of SEQ ID NO: 70, or an amino acid sequence having at least 80% identity, preferably at least 85% identity, more preferably 90% or 95% identity to the amino acid sequence of SEQ ID NO: 70; 8) the α chain variable region comprises CDR1 comprising the amino acid sequence of SEQ ID NO: 73, CDR2 comprising the amino acid sequence of SEQ ID NO: 74, and CDR3 comprising the amino acid sequence of SEQ ID NO: 75, or an amino acid sequence having at least 80% identity, preferably at least 85% identity, more preferably 90% or 95% identity to the amino acid sequence of SEQ ID NO: 75; the β chain variable region comprises CDR1 comprising the amino acid sequence of SEQ ID NO: 78, CDR2 comprising the amino acid sequence of SEQ ID NO: 79, and CDR3 comprising the amino acid sequence of SEQ ID NO: 80, or an amino acid sequence having at least 80% identity, preferably at least 85% identity, more preferably 90% or 95% identity to the amino acid sequence of SEQ ID NO: 80; or 9) the α chain variable region comprises CDR1 comprising the amino acid sequence of SEQ ID NO: 83, CDR2 comprising the amino acid sequence of SEQ ID NO: 84, and CDR3 comprising the amino acid sequence of SEQ ID NO: 85, or an amino acid sequence having at least 80% identity, preferably at least 85% identity, more preferably 90% or 95% identity to the amino acid sequence of SEQ ID NO: 85; the β chain variable region comprises CDR1 comprising the amino acid sequence of SEQ ID NO: 88, CDR2 comprising the amino acid sequence of SEQ ID NO: 89, and CDR3 comprising the amino acid sequence of SEQ ID NO: 90, or an amino acid sequence having at least 80% identity, preferably at least 85% identity, more preferably 90% or 95% identity to the amino acid sequence of SEQ ID NO: 90.

In some embodiments, in the binding protein: 1) the α chain variable region comprises the amino acid sequence of SEQ ID NO: 2 or an amino acid sequence having at least 80% identity, preferably at least 85% identity, more preferably 90% or 95% identity to the amino acid sequence of SEQ ID NO: 2; the β chain variable region comprises the amino acid sequence of SEQ ID NO: 7 or an amino acid sequence having at least 80% identity, preferably at least 85% identity, more preferably 90% or 95% identity to the amino acid sequence of SEQ ID NO: 7; 2) the α chain variable region comprises the amino acid sequence of SEQ ID NO: 12 or an amino acid sequence having at least 80% identity, preferably at least 85% identity, more preferably 90% or 95% identity to the amino acid sequence of SEQ ID NO: 12; the β chain variable region comprises the amino acid sequence of SEQ ID NO: 17 or an amino acid sequence having at least 80% identity, preferably at least 85% identity, more preferably 90% or 95% identity to the amino acid sequence of SEQ ID NO: 17; 3) the α chain variable region comprises the amino acid sequence of SEQ ID NO: 22 or an amino acid sequence having at least 80% identity, preferably at least 85% identity, more preferably 90% or 95% identity to the amino acid sequence of SEQ ID NO: 22; the β chain variable region comprises the amino acid sequence of SEQ ID NO: 27 or an amino acid sequence having at least 80% identity, preferably at least 85% identity, more preferably 90% or 95% identity to the amino acid sequence of SEQ ID NO: 27; 4) the α chain variable region comprises the amino acid sequence of SEQ ID NO: 32 or an amino acid sequence having at least 80% identity, preferably at least 85% identity, more preferably 90% or 95% identity to the amino acid sequence of SEQ ID NO: 32; the β chain variable region comprises the amino acid sequence of SEQ ID NO: 37 or an amino acid sequence having at least 80% identity, preferably at least 85% identity, more preferably 90% or 95% identity to the amino acid sequence of SEQ ID NO: 37; 5) the α chain variable region comprises the amino acid sequence of SEQ ID NO: 42 or an amino acid sequence having at least 80% identity, preferably at least 85% identity, more preferably 90% or 95% identity to the amino acid sequence of SEQ ID NO: 42; the β chain variable region comprises the amino acid sequence of SEQ ID NO: 47 or an amino acid sequence having at least 80% identity, preferably at least 85% identity, more preferably 90% or 95% identity to the amino acid sequence of SEQ ID NO: 47; 6) the α chain variable region comprises the amino acid sequence of SEQ ID NO: 52 or an amino acid sequence having at least 80% identity, preferably at least 85% identity, more preferably 90% or 95% identity to the amino acid sequence of SEQ ID NO: 52; the β chain variable region comprises the amino acid sequence of SEQ ID NO: 57 or an amino acid sequence having at least 80% identity, preferably at least 85% identity, more preferably 90% or 95% identity to the amino acid sequence of SEQ ID NO: 57; 7) the α chain variable region comprises the amino acid sequence of SEQ ID NO: 62 or an amino acid sequence having at least 80% identity, preferably at least 85% identity, more preferably 90% or 95% identity to the amino acid sequence of SEQ ID NO: 62; the β chain variable region comprises the amino acid sequence of SEQ ID NO: 67 or an amino acid sequence having at least 80% identity, preferably at least 85% identity, more preferably 90% or 95% identity to the amino acid sequence of SEQ ID NO: 67; 8) the α chain variable region comprises the amino acid sequence of SEQ ID NO: 72 or an amino acid sequence having at least 80% identity, preferably at least 85% identity, more preferably 90% or 95% identity to the amino acid sequence of SEQ ID NO: 72; the β chain variable region comprises the amino acid sequence of SEQ ID NO: 77 or an amino acid sequence having at least 80% identity, preferably at least 85% identity, more preferably 90% or 95% identity to the amino acid sequence of SEQ ID NO: 77; or 9) the α chain variable region comprises the amino acid sequence of SEQ ID NO: 82 or an amino acid sequence having at least 80% identity, preferably at least 85% identity, more preferably 90% or 95% identity to the amino acid sequence of SEQ ID NO: 82; the β chain variable region comprises the amino acid sequence of SEQ ID NO: 87 or an amino acid sequence having at least 80% identity, preferably at least 85% identity, more preferably 90% or 95% identity to the amino acid sequence of SEQ ID NO: 87.

In some embodiments, the binding protein comprises the TCR α chain and TCR β chain, wherein: 1) the α chain comprises the amino acid sequence of SEQ ID NO: 1 or an amino acid sequence having at least 80% identity, preferably at least 85% identity, more preferably 90% or 95% identity to the amino acid sequence of SEQ ID NO: 1; the β chain comprises the amino acid sequence of SEQ ID NO: 6 or an amino acid sequence having at least 80% identity, preferably at least 85% identity, more preferably 90% or 95% identity to the amino acid sequence of SEQ ID NO: 6; 2) the α chain comprises the amino acid sequence of SEQ ID NO: 11 or an amino acid sequence having at least 80% identity, preferably at least 85% identity, more preferably 90% or 95% identity to the amino acid sequence of SEQ ID NO: 11; the β chain comprises the amino acid sequence of SEQ ID NO: 16 or an amino acid sequence having at least 80% identity, preferably at least 85% identity, more preferably 90% or 95% identity to the amino acid sequence of SEQ ID NO: 16; 3) the α chain comprises the amino acid sequence of SEQ ID NO: 21 or an amino acid sequence having at least 80% identity, preferably at least 85% identity, more preferably 90% or 95% identity to the amino acid sequence of SEQ ID NO: 21; the β chain comprises the amino acid sequence of SEQ ID NO: 26 or an amino acid sequence having at least 80% identity, preferably at least 85% identity, more preferably 90% or 95% identity to the amino acid sequence of SEQ ID NO: 26; 4) the α chain comprises the amino acid sequence of SEQ ID NO: 31 or an amino acid sequence having at least 80% identity, preferably at least 85% identity, more preferably 90% or 95% identity to the amino acid sequence of SEQ ID NO: 31; the β chain comprises the amino acid sequence of SEQ ID NO: 36 or an amino acid sequence having at least 80% identity, preferably at least 85% identity, more preferably 90% or 95% identity to the amino acid sequence of SEQ ID NO: 36; 5) the α chain comprises the amino acid sequence of SEQ ID NO: 41 or an amino acid sequence having at least 80% identity, preferably at least 85% identity, more preferably 90% or 95% identity to the amino acid sequence of SEQ ID NO: 41; the β chain comprises the amino acid sequence of SEQ ID NO: 46 or an amino acid sequence having at least 80% identity, preferably at least 85% identity, more preferably 90% or 95% identity to the amino acid sequence of SEQ ID NO: 46; 6) the α chain comprises the amino acid sequence of SEQ ID NO: 51 or an amino acid sequence having at least 80% identity, preferably at least 85% identity, more preferably 90% or 95% identity to the amino acid sequence of SEQ ID NO: 51; the β chain comprises the amino acid sequence of SEQ ID NO: 56 or an amino acid sequence having at least 80% identity, preferably at least 85% identity, more preferably 90% or 95% identity to the amino acid sequence of SEQ ID NO: 56; 7) the α chain comprises the amino acid sequence of SEQ ID NO: 61 or an amino acid sequence having at least 80% identity, preferably at least 85% identity, more preferably 90% or 95% identity to the amino acid sequence of SEQ ID NO: 61; the β chain comprises the amino acid sequence of SEQ ID NO: 66 or an amino acid sequence having at least 80% identity, preferably at least 85% identity, more preferably 90% or 95% identity to the amino acid sequence of SEQ ID NO: 66; 8) the α chain comprises the amino acid sequence of SEQ ID NO: 71 or an amino acid sequence having at least 80% identity, preferably at least 85% identity, more preferably 90% or 95% identity to the amino acid sequence of SEQ ID NO: 71; the β chain comprises the amino acid sequence of SEQ ID NO: 76 or an amino acid sequence having at least 80% identity, preferably at least 85% identity, more preferably 90% or 95% identity to the amino acid sequence of SEQ ID NO: 76; or 9) the α chain comprises the amino acid sequence of SEQ ID NO: 81 or an amino acid sequence having at least 80% identity, preferably at least 85% identity, more preferably 90% or 95% identity to the amino acid sequence of SEQ ID NO: 81; the β chain comprises the amino acid sequence of SEQ ID NO: 86 or an amino acid sequence having at least 80% identity, preferably at least 85% identity, more preferably 90% or 95% identity to the amino acid sequence of SEQ ID NO: 86.

In some embodiments, the binding protein is a protein in the form of:
1) a TCR molecule;
2) an antibody molecule; or
3) a CAR molecule.

In some embodiments, when T cells expressing the binding protein are co-incubated with HLA-A*11: 01 cells in the presence of the antigen peptide, or when T cells expressing the binding protein are co-incubated with HLA-A*11:01 cells expressing the antigen peptide, the T cells specifically kill the HLA-A*11:01 cells.

In some embodiments, when T cells expressing the binding protein are co-incubated with HLA-A*11:01 cells in the presence of the antigen peptide, or when T cells expressing the binding protein are co-incubated with HLA-A*11:01 cells expressing the antigen peptide, the T cells produce IFN-γ and/or express CD69, or exhibit increased production of IFN-γ and/or expression of CD69.

In some embodiments, the binding protein further comprises a conjugate covalently or non-covalently linked to the binding domain; preferably, the conjugate is a detectable label, a radioisotope, or a therapeutic agent.

In another aspect, the present invention provides an isolated nucleic acid molecule encoding the binding protein, the α chain variable region, the β chain variable region, the α chain, or the β chain.

In another aspect, the present invention provides a vector comprising the nucleic acid molecule.

In another aspect, the present invention provides a host cell expressing the binding protein, or comprising the nucleic acid molecule or the vector.

In some embodiments, the host cell is a mammalian cell, preferably a human cell.

In some embodiments, the host cell is a T cell or an NK cell.

In some embodiments, the host cell exhibits cytotoxic activity against HLA-A*11:01 cells expressing the antigen peptide VVGAVGVGK (SEQ ID NO: 91) or VVVGAVGVGK (SEQ ID NO: 92).

In another aspect, the present invention provides a pharmaceutical composition comprising: 1) the binding protein, the nucleic acid molecule, the vector, or the host cell; and 2) a pharmaceutically acceptable carrier.

In another aspect, the present invention provides the use of the binding protein, the nucleic acid molecule, the vector, or the host cell in the manufacture of a medicament for treating a tumor, wherein the tumor expresses the antigen peptide.

In another aspect, the present invention provides a method for treating a tumor in a subject, comprising administering to the subject a therapeutically effective amount of the binding protein, the nucleic acid molecule, the vector, the host cell, or the pharmaceutical composition.

In some embodiments, the tumor is selected from pancreatic cancer, colorectal cancer, lung cancer, cholangiocarcinoma, endometrial cancer, and ovarian cancer.

In another aspect, the present invention provides a detection kit comprising the binding protein.

### Description of drawings

Figure 1 shows a representative flow cytometry schematic depicting the pentamer enrichment and sorting of HLA-A*11:01/KRAS G12V-specific T cells after stimulation.
Figure 2A shows the cytotoxic activity of untransduced and HLA-A*11:01/KRAS G12V-specific TCR-transduced CD8⁺ T cells against K562-A*11:01 target cells loaded with different antigen peptides. The peptides loaded are: G12V 9-mer peptide: VVGAVGVGK (SEQ ID NO: 91), G12V 10-mer peptide: VVVGAVGVGK (SEQ ID NO: 92), and WT peptide: VVGAGGVGK (SEQ ID NO: 93) + VVVGAGGVGK (SEQ ID NO: 94).
Figure 2B shows the cytotoxic activity of untransduced and HLA-A*11:01/KRAS G12V-specific TCR-transduced CD8⁺ T cells against BxPC3 cells overexpressing HLA-A*11:01 and/or KRAS G12V. BxPC3-A1101 is BxPC3 cell transduced with HLA-A*11:01, BxPC3-A1101-G12V is BxPC3 cell co-transduced with HLA-A*11:01 and KRAS G12V, and BxPC3-A1101-G12D is BxPC3 cell co-transduced with HLA-A*11:01 and KRAS G12D.
Figure 3 shows IFN-γ release by TCR1-transduced T cells after co-culture with peptide-loaded target cells. BxPC3-A1101 is BxPC3 cell transduced with HLA-A*11:01; BxPC3-G12V is BxPC3 cell transduced with KRAS G12V; BxPC3-A1101-G12V is BxPC3 cell co-transduced with both HLA-A*11:01 and KRAS G12V.
Figure 4A shows IFN-γ release by specific TCR-transduced PBMC detected by ELISPOT assay after co-cultured with target cells (K562-A*11:01) loaded with peptides at graded concentrations (10⁻¹¹ to 10⁻⁶ M).
Figure 4B shows CD69 expression of specific TCR-transduced Jurkat cells detected by flow cytometry after co-culture with target cells (K562-A*11:01) loaded with peptides at graded concentrations (10⁻¹¹to 10⁻⁶ M).
Figure 4C shows CD69 expression of specific TCR-transduced Jurkat cells detected by flow cytometry after co-culture with target cells (K562-A*11:01) loaded with either G12V-9 or G12V-10 peptides at graded concentrations (10⁻¹¹ to 10⁻⁶ M).

### Detailed Description

Unless specifically indicated otherwise, all technical and scientific terms used herein have the same meaning as commonly understood by those of ordinary skill in the art to which this invention belongs.

The term "or" refers to a single element among the listed alternatives, unless the context clearly indicates otherwise. The term "and/or" refers to any one, any two, any three, any more, or all of the listed alternative elements.

The term "about" generally refers to a variation within ±10% of the specified value, such as within ±0.5%, ±1%, ±1.5%, ±2%, ±2.5%, ±3%, ±3.5%, ±4%, ±4.5%, ±5%, ±5.5%, ±6%, ±6.5%, ±7%, ±7.5%, ±8%, ±8.5%, ±9%, +9.5%, or ±10% of the specified value.

The terms "comprise" or "comprising" mean that the recited elements, integers, or steps are included, but do not exclude any other elements, integers, or steps. As used herein, unless otherwise indicated, the terms "comprise" or "comprising" also encompass embodiments "consisting of" the recited elements, integers, or steps. For example, when referring to a variable region "comprising" a specific sequence, it is also intended to encompass a variable region "consisting of" the specific sequence.

The term "Major Histocompatibility Complex (MHC)" refers to a group of genes collectively encoding major histocompatibility antigens in animals. Currently, the structures of MHC Class I and Class II molecules have been fully resolved, both belonging to the immunoglobulin superfamily. Human MHC is designated HLA (Human Leukocyte Antigen). HLA Class I molecules primarily comprise protein products (heavy chains or α-chains) of three functional genes: *HLA-A, HLA-B,* and *HLA-C.* Each HLA Class I heavy chain pairs with β2-microglobulin (β2m) to form a complete MHC Class I molecule as a heterodimer. Due to the polymorphism of HLA-encoding genes, the HLA Nomenclature Committee has established naming principles for specific alleles, the key points of which include: for a given allele, the three-letter HLA prefix is followed by the locus name connected by a hyphen (e.g., *HLA-A);* an asterisk (*) is added after the locus name, followed by the numerical identifier of the allele's gene family (corresponding to serological typing where possible); a colon (:) follows the gene family number, preceding the specific allele number (e.g., *HLA-A*02:01*). One primary function of MHC molecules is antigen presentation, whereby MHC molecules bind antigen peptides via their peptide-binding groove to form peptide-MHC complexes (pMHC), which are presented on the cell surface for recognition by T cells (via TCR) to trigger immune responses.

The term "antigen peptide" refers to a short peptide capable of binding to the peptide-binding groove of an MHC molecule (or HLA molecule). In HLA Class I molecules, the antigen-binding groove is formed by the α1 and α2 domains of the heavy chain (whose extracellular region comprises three domains: α1, α2, and α3). Each domain folds into one α-helix and four β-sheets. The two α-helices constitute the walls of the groove, while the eight β-sheets form its floor. The length of antigen peptides suitable for accommodation in this binding groove is typically 8-11 amino acids. In a specific embodiment, the antigen peptide is a mutant KRAS protein fragment: VVGAVGVGK (SEQ ID NO: 91). In another specific embodiment, the antigen peptide is a mutant KRAS protein fragment: VVVGAVGVGK (SEQ ID NO: 92).

The terms "antigen peptide:MHC complex" or "antigen peptide:HLA complex" refer to a complex (pMHC) formed by an MHC molecule and an antigen peptide, wherein the antigen peptide resides within the peptide-binding groove. TCR molecule recognizes the complex viCDR sequences in its binding domain and is expected to bind specifically thereto. Binding of the TCR molecule to the pMHC complex is MHC-dependent, meaning the TCR molecule recognizes both the antigen peptide and the pMHC complex concurrently.

The term "T cell receptor (TCR)" refers to the functional unit of T lymphocytes for antigen (pMHC) recognition, belonging to the immunoglobulin superfamily. The TCR expressed on T cells is a cell-surface glycoprotein existing as a heterodimer of either α chain/β chain or γ chain/δ chain. In humans, the vast majority (90%-95%) of TCRs in peripheral blood are heterodimers composed of α chain and β chain. Both the α chain and β chain comprise a variable region (Vα, Vβ), a constant region (Cα, Cβ), a transmembrane region, and a cytoplasmic region. The cytoplasmic regions of α chain and β chain are short, containing only several amino acids. The constant regions (Cα, Cβ) exhibit relatively limited sequence variation, with specific amino acid sequences being publicly accessible in databases such as the International ImMunoGeneTics Information System (IMGT). The transmembrane region comprises positively charged amino acid residues that non-covalently interact with negatively charged residues in the transmembrane regions of CD3 molecules, thereby stabilizing the TCR-CD3 complex structure. Analysis of amino acid sequences in TCR variable regions reveals three hypervariable regions in each of Vα and Vβ, designated complementarity-determining regions (CDRs) CDR1, CDR2, and CDR3. CDR3 exhibits the greatest variability and largely determines the antigen specificity of the TCR. Analogous to antibody molecules, the variable regions further comprise four framework regions (FR1-FR4) flanking the CDRs. The positional definitions of CDR1-CDR3 and FR1-FR4 within the full-length TCR sequence are established according to the IMGT nomenclature, which is well-known and publicly available in IMGT databases. The variable regions of the α chain and β chain, particularly the three CDRs in the α chain and the three CDRs in the β chain, collectively form the portion of the TCR that specifically recognizes and binds to the pMHC complex (referred to herein as the "binding domain"). In the immune system, binding of an antigen-specific TCR to a pMHC complex initiates direct physical contact between the T cell and an antigen-presenting cell (APC) or target cell, leading to T cell activation or cytotoxic activity against the target cell. Unless the context otherwise dictates or implies, the term "TCR" as used herein further encompasses functional antigen-specific fragments thereof, such as Vα and Vβ variable regions linked by a short peptide linker.

The term "antibody" is used herein in its broadest sense and includes immunoglobulins or other molecular entities comprising one or more antigen-binding domains that specifically bind to an antigen, referring to proteins or polypeptides exhibiting binding specificity for a particular antigen. Specific examples of antibodies may include intact antibodies (e.g., classical four-chain antibody molecules), single-chain antibodies, single-domain antibodies, multi-specific antibodies, and the like. Classical antibody molecules typically comprise tetramers formed by two identical heavy chains and two identical light chains interconnected via disulfide bonds. Based on differences in amino acid sequence conservation, both heavy and light chains comprise an amino-terminal variable region (V) and a carboxy-terminal constant region (C). The variable region mediates antigen recognition and binding, while the constant region (e.g., Fc fragment) initiates downstream effector functions such as antibody-dependent cell-mediated cytotoxicity (ADCC). Within the variable regions of the heavy and light chains, three localized segments exhibit heightened variability in amino acid composition and sequence, constituting key sites for antigen binding and thus designated complementarity-determining regions (CDRs). The three CDRs of the heavy chain are designated HCDR1, HCDR2, and HCDR3, while those of the light chain are designated LCDR1, LCDR2, and LCDR3. Each heavy chain variable region (VH) and light chain variable region (VL) may comprise three CDRs and four framework regions (FRs), arranged from amino-terminus to carboxy-terminus in the order of: FR1, CDR1, FR2, CDR2, FR3, CDR3, and FR4. As used herein, antibody molecules further encompass fusion proteins formed by recombinant fusion of a TCR molecule's variable region with an antibody molecule's constant region, which simultaneously possess the antigen specificity of the TCR molecule and the effector functions of the antibody constant region.

The term "Chimeric Antigen Receptor (CAR)" refers to an engineered transmembrane receptor molecule that confers desired specificity to immune effector cells, such as the ability of binding to a specific tumor antigen. A CAR typically comprises an extracellular antigen-binding domain, a transmembrane domain, and an intracellular signaling domain. In some instances, the antigen-binding domain is a single-chain variable fragment (scFv) or single-domain antibody fragment responsible for recognizing and binding to a specific antigen. The intracellular signaling domain generally includes immunoreceptor tyrosine-based activation motif (ITAM), such as a signaling domain derived from CD3ζ, which activates immune effector cells to exert cytotoxic effects. Additionally, the CAR may comprise an N-terminal signal peptide for intracellular trafficking of the nascent protein and a hinge region between the antigen-binding domain and transmembrane domain. Besides the signaling domain, the intracellular domain may further include a co-stimulatory domain derived from molecules such as 4-1BB or CD28.

The terms "antigen-specific," "targeting," or "specifically binds to" refer to the property whereby a TCR (or other fusion protein comprising its binding domain, e.g., CAR) specifically recognizes and binds its corresponding pMHC complex with high affinity. Relative to other molecules present in the environment, the TCR (or fusion protein comprising the binding domain thereof, such as CAR) exhibits higher binding affinity for its antigen-specific target molecule (e.g., pMHC complex). Binding affinity of the TCR for the pMHC complex may be measured by parameters such as EC₅₀ or KD values.

The term "binding protein" refers to a protein capable of specifically binding to a particular ligand (e.g., an antigen). As used herein, binding proteins encompass: TCR molecules (including single-chain TCRs and soluble TCRs) with specificity for pMHC complexes, fusion proteins thereof with other proteins (e.g., antibody molecules or CARs); and immunoconjugates (conjugated with detectable labels, therapeutic agents, or radioisotopes).

The term "fusion protein" denotes an artificially generated protein (e.g., via genetic engineering techniques) comprising at least two heterologous peptide segments not naturally occurring together in a single polypeptide. Representative examples include multi-specific antibodies, antibody-cytokine fusion proteins, enzyme-conjugated antibodies for immunoassays, and chimeric antigen receptors (CARs).

The term "variant" as applied to a TCR (or its antigen-specific fragment) refers to a protein derived from a parent TCR (or fragment) by introduction of one or more amino acid insertions, deletions, or substitutions, while retaining at least partial functionality of the parent molecule (particularly the function of interest, e.g., binding capacity to the corresponding pMHC complex). For instance, such variants of TCR (or the antigen-specific fragment thereof) may retain at least 20%, 30%, 40%, 50%, 60%, 70%, 80%, or 90% of the parent TCR's binding capacity to pMHC, or even exhibit enhanced binding capacity. In some embodiments, variants of TCR (or the antigen-specific fragment thereof) retain at least 80%, 85%, 90%, 95%, or 100% (or greater) of the parent TCR's antigen-binding affinity. Variants of TCR (or the antigen-specific fragment thereof) typically comprise amino acid modifications in framework and/or constant regions but may include limited substitutions in CDR sequences. Accordingly, those skilled in the art will appreciate that variants of the disclosed TCRs (or fragments) may be obtained by introducing minor substitutions, deletions, or additions into the provided amino acid sequences (CDRs, variable regions, orα/β chains), followed by verification of pMHC binding capacity or biological activity, all such variants being encompassed within the scope of the present invention.

Herein, the terms "nucleic acid molecule", "nucleic acid" and "polynucleotide" are interchangeably used and refer to nucleotide polymers. Such nucleotide polymers may contain natural and/ or non-natural nucleotides and include, but are not limited to, DNA, RNA, and PNA. The "nucleic acid sequence" refers to a linear sequence of nucleotides contained in a nucleic acid molecule or polynucleotide.

The term "vector" refers to a nucleic acid molecule that can be engineered to contain a polynucleotide of interest (e.g., a coding sequence of a polypeptide of interest), or a nucleic acid molecule that can be replicated in a host cell (e.g., a nucleic acid, plasmid, or virus, etc.). The vector may include one or more of the following components: an origin of replication, one or more regulatory sequences that regulate the expression of the polynucleotide of interest (such as a promoter and/or an enhancer), and/or one or more selectable marker genes (such as antibiotic resistance genes and genes that can be used in colorimetric analysis, e.g., β-galactose). The term "expression vector" refers to a vector used to express a polypeptide of interest in a host cell.

The "host cell" refers to cells which may be or have been vectors or receptors of isolated polynucleotides. The host cell may be a prokaryotic cell or a eukaryotic cell. Exemplary eukaryotic cells include mammalian cells, such as primate or non-primate cells; fungal cells, such as yeast; plant cells; and insect cells. Non-limiting exemplary mammalian cells include (but are not limited to) CHO cells, HEK-293 cells, BHK cells or PER-C6 cells, and derived cells thereof, such as 293-6E, CHO-DG44, CHO-K1, CHO-S and CHO-DS cells. In some embodiments, host cells are utilized for producing target proteins; for instance, the TCR α and/or β chains provided herein can be secreted by mammalian cells. In other embodiments, host cells exhibit specific functions after expressing the TCR molecules described herein-such as T cells acquiring the ability to recognize and eliminate target cells presenting specific antigen peptides upon expressing the provided TCR molecules. Host cells may exist as isolated cells or cell lines, and also encompass cells transfected *in vivo* with the nucleic acid molecules or the expression vectors provided herein. Host cells include the progeny of a single host cell, and the progeny may not necessarily be completely identical to the original parent cell (in morphology or genomic DNA complementarity) due to natural, accidental, or deliberate mutation.

The "subject" refers to animals, such as mammals, including but not limited to humans, rodents, apes, feline, canine, equine, bovine, swine, sheep, goats, mammalian laboratory animals, mammalian livestock, mammalian sports animals, and mammalian pets. The subject may be male or female and may be of any appropriate age, including infants, young children, adolescents, adults, and elderly subjects. In some embodiments, the subject refers to an individual in need of treatment for a disease or disorder. In some embodiments, the subject receiving treatment may be a patient who suffers from, or is at risk of developing, a disorder associated with the treatment. In certain embodiments, the subject is a human, such as a human patient. The term is often used interchangeably with a "patient", a "detection subject", a "treatment subject", etc.

When referring to pharmaceutical compositions, the term "pharmaceutically acceptable carrier" used refers to substances such as solid or liquid diluents, fillers, antioxidants, and stabilizers and the like, which can be safe for administration, and which are suitable for administration to humans and/or animals without undue adverse side effects, while being suitable for maintaining the activity of the drug or active agent therein.

When referring to disease treatment, the "effective amount" refers to an amount of an active compound sufficient to elicit the biological or medical response desired by a clinician in a subject. The "therapeutically effective amount" of the fusion protein of the present invention can be determined by those skilled in the art according to the administration route, the subject's body weight, age or condition, and other factors. For example, a typical daily dose may range from 0.01 mg to 100 mg or more of active ingredient per kg of body weight. For immune effector cells (e.g., T cells) expressing the TCR provided herein, the number of cells administered each infusion may range, for example, from approximately 1×10⁶ to approximately 1×10¹² cells or higher. In certain embodiments, fewer than 1×10⁶ T cells may be administered. Administration methods for the active molecules (e.g., TCR, antibodies, fusion proteins) or immune effector cells provided herein include, but are not limited to, injection, such as intravenous, intramuscular, intra-arterial, subcutaneous, intraperitoneal, and the like.

When referring to amino acid or nucleotide sequences, the term "sequence identity" (also known as sequence consistency) when referring to amino acid or nucleotide sequences refers to the degree of identity between two amino acid or nucleotide sequences (e.g., a query sequence and a reference sequence), usually expressed as a percentage. Typically, before calculating the identity percentage between two amino acid or nucleotide sequences, the sequence alignment is first performed, and a gap (if any) is introduced. If at a certain alignment position, the amino acid residues or bases in the two sequences are the same, the two sequences are considered to be identical or matched at the position; and if the amino acid residues or bases in the two sequences are different, they are considered to be non-identical or mismatched at the position. In some algorithms, the number of matched positions is divided by the total number of positions in the alignment window to obtain sequence identity. In other algorithms, the numbers of gaps and/or the gap length are also taken into account. For the purposes of the present invention, the published alignment software BLAST (available at ncbi.nlm.nih.gov) can be employed to obtain optimal sequence alignments by using default settings, and calculate the sequence identity between two amino acid or nucleotide sequences. in some embodiments, the expression "at least 80% sequence identity" as used herein includes, but is not limited to, cases of at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or even 100% sequence identity.

The present disclosure provides isolated or purified binding proteins (e.g., T cell receptors (TCRs), antibodies, or chimeric antigen receptors (CARs)) that are antigen-specific for a peptide-MHC complex, wherein the antigen peptide comprises amino acid mutations relative to the wild-type sequence and is derived from a mutated KRAS protein.

The present disclosure also provides polypeptides and proteins associated with the TCR or its binding domain, along with related nucleic acid molecules, vectors (e.g., recombinant expression vectors), host cells (e.g., T cells), pharmaceutical compositions, and the like.

The present disclosure further provides methods and kits for detecting the presence of tumors in a subject, as well as methods for treating or preventing tumors in a subject.

### T cell receptor (TCR)

The present disclosure provides an isolated or purified T cell receptor (TCR) having antigen specificity for a peptide-MHC complex, wherein the peptide comprises amino acid mutations relative to a wild-type sequence and is derived from a mutated KRAS protein, and comprises (1) VVGAVGVGK (SEQ ID NO: 91) or (2) VVVGAVGVGK (SEQ ID NO: 92), or consisting thereof. Considering that MHC class I molecules do not strictly limit the length of peptides, the inventors expect that one or two amino acids may be added to or deleted from (particularly at terminus of) the peptide sequences provided herein, and the binding proteins or TCR molecules provided herein also have binding capability for these peptide variant-MHC complexes.

The TCR provided herein may be in αβ heterodimeric form or single-chain form (scTCR). Examples of single-chain forms may include αβ TCR polypeptides in Vα-L-Vβ, Vβ-L-Vα, Vα-Cα-L-Vβ, or Vα-L-Vβ-Cβ forms (wherein Vα and Vβ refer to the variable regions of the TCR α chain and TCR β chain, respectively; Cα and Cβ refer to the constant regions of the TCR α chain and TCR β chain, respectively; and L is a linker sequence, typically a short peptide). The linker sequence may be conventional in the art and is frequently used in antibody engineering and TCR engineering. The TCR provided herein may also be a water-soluble TCR molecule lacking transmembrane regions of the α and β chains.

In some embodiments, the present disclosure provides a TCR molecule having antigen specificity for the peptide VVGAVGVGK (SEQ ID NO: 91) -MHC complex or VVVGAVGVGK (SEQ ID NO: 92) -MHC complex. The CDR3 of the α chain variable region of the TCR molecule comprises the amino acid sequence of SEQ ID NO: 5, 15, 25, 35, 45, 55, 65, 75, or 85, or an amino acid sequence having at least 80% identity, preferably at least 85% identity, more preferably 90% or 95% identity to the amino acid sequence of SEQ ID NO: 5, 15, 25, 35, 45, 55, 65, 75, or 85; the CDR3 of the β chain variable region of the TCR molecule comprises the amino acid sequence of SEQ ID NO: 10, 20, 30, 40, 50, 60, 70, 80, or 90, or an amino acid sequence having at least 80% identity, preferably at least 85% identity, more preferably 90% or 95% identity to the amino acid sequence of SEQ ID NO: 10, 20, 30, 40, 50, 60, 70, 80, or 90. Preferably, the MHC molecule is HLA-A*11:01.

Further, the α chain variable region comprises CDR1 comprising the amino acid sequence of SEQ ID NO: 3, CDR2 comprising the amino acid sequence of SEQ ID NO: 4, and CDR3 comprising the amino acid sequence of SEQ ID NO: 5, or an amino acid sequence having at least 80% identity, preferably at least 85% identity, more preferably 90% or 95% identity to the amino acid sequence of SEQ ID NO: 5; CDR1 comprising the amino acid sequence of SEQ ID NO: 13, CDR2 comprising the amino acid sequence of SEQ ID NO: 14, and CDR3 comprising the amino acid sequence of SEQ ID NO: 15, or an amino acid sequence having at least 80% identity, preferably at least 85% identity, more preferably 90% or 95% identity to the amino acid sequence of SEQ ID NO: 15; CDR1 comprising the amino acid sequence of SEQ ID NO: 23, CDR2 comprising the amino acid sequence of SEQ ID NO: 24, and CDR3 comprising the amino acid sequence of SEQ ID NO: 25, or an amino acid sequence having at least 80% identity, preferably at least 85% identity, more preferably 90% or 95% identity to the amino acid sequence of SEQ ID NO: 25; CDR1 comprising the amino acid sequence of SEQ ID NO: 33, CDR2 comprising the amino acid sequence of SEQ ID NO: 34, and CDR3 comprising the amino acid sequence of SEQ ID NO: 35, or an amino acid sequence having at least 80% identity, preferably at least 85% identity, more preferably 90% or 95% identity to the amino acid sequence of SEQ ID NO: 35; CDR1 comprising the amino acid sequence of SEQ ID NO: 43, CDR2 comprising the amino acid sequence of SEQ ID NO: 44, and CDR3 comprising the amino acid sequence of SEQ ID NO: 45, or an amino acid sequence having at least 80% identity, preferably at least 85% identity, more preferably 90% or 95% identity to the amino acid sequence of SEQ ID NO: 45; CDR1 comprising the amino acid sequence of SEQ ID NO: 53, CDR2 comprising the amino acid sequence of SEQ ID NO: 54, and CDR3 comprising the amino acid sequence of SEQ ID NO: 55, or an amino acid sequence having at least 80% identity, preferably at least 85% identity, more preferably 90% or 95% identity to the amino acid sequence of SEQ ID NO: 55; CDR1 comprising the amino acid sequence of SEQ ID NO: 63, CDR2 comprising the amino acid sequence of SEQ ID NO: 64, and CDR3 comprising the amino acid sequence of SEQ ID NO: 65, or an amino acid sequence having at least 80% identity, preferably at least 85% identity, more preferably 90% or 95% identity to the amino acid sequence of SEQ ID NO: 65; CDR1 comprising the amino acid sequence of SEQ ID NO: 73, CDR2 comprising the amino acid sequence of SEQ ID NO: 74, and CDR3 comprising the amino acid sequence of SEQ ID NO: 75, or an amino acid sequence having at least 80% identity, preferably at least 85% identity, more preferably 90% or 95% identity to the amino acid sequence of SEQ ID NO: 75; or CDR1 comprising the amino acid sequence of SEQ ID NO: 83, CDR2 comprising the amino acid sequence of SEQ ID NO: 84, and CDR3 comprising the amino acid sequence of SEQ ID NO: 85, or an amino acid sequence having at least 80% identity, preferably at least 85% identity, more preferably 90% or 95% identity to the amino acid sequence of SEQ ID NO: 85, the β chain variable region comprises CDR1 comprising the amino acid sequence of SEQ ID NO: 8, CDR2 comprising the amino acid sequence of SEQ ID NO: 9, and CDR3 comprising the amino acid sequence of SEQ ID NO: 10, or an amino acid sequence having at least 80% identity, preferably at least 85% identity, more preferably 90% or 95% identity to the amino acid sequence of SEQ ID NO: 10; CDR1 comprising the amino acid sequence of SEQ ID NO: 18, CDR2 comprising the amino acid sequence of SEQ ID NO: 19, and CDR3 comprising the amino acid sequence of SEQ ID NO: 20, or an amino acid sequence having at least 80% identity, preferably at least 85% identity, more preferably 90% or 95% identity to the amino acid sequence of SEQ ID NO: 20; CDR1 comprising the amino acid sequence of SEQ ID NO: 28, CDR2 comprising the amino acid sequence of SEQ ID NO: 29, and CDR3 comprising the amino acid sequence of SEQ ID NO: 30, or an amino acid sequence having at least 80% identity, preferably at least 85% identity, more preferably 90% or 95% identity to the amino acid sequence of SEQ ID NO: 30; CDR1 comprising the amino acid sequence of SEQ ID NO: 38, CDR2 comprising the amino acid sequence of SEQ ID NO: 39, and CDR3 comprising the amino acid sequence of SEQ ID NO: 40, or an amino acid sequence having at least 80% identity, preferably at least 85% identity, more preferably 90% or 95% identity to the amino acid sequence of SEQ ID NO: 40; CDR1 comprising the amino acid sequence of SEQ ID NO: 48, CDR2 comprising the amino acid sequence of SEQ ID NO: 49, and CDR3 comprising the amino acid sequence of SEQ ID NO: 50, or an amino acid sequence having at least 80% identity, preferably at least 85% identity, more preferably 90% or 95% identity to the amino acid sequence of SEQ ID NO: 50; CDR1 comprising the amino acid sequence of SEQ ID NO: 58, CDR2 comprising the amino acid sequence of SEQ ID NO: 59, and CDR3 comprising the amino acid sequence of SEQ ID NO: 60, or an amino acid sequence having at least 80% identity, preferably at least 85% identity, more preferably 90% or 95% identity to the amino acid sequence of SEQ ID NO: 60; CDR1 comprising the amino acid sequence of SEQ ID NO: 68, CDR2 comprising the amino acid sequence of SEQ ID NO: 69, and CDR3 comprising the amino acid sequence of SEQ ID NO: 70, or an amino acid sequence having at least 80% identity, preferably at least 85% identity, more preferably 90% or 95% identity to the amino acid sequence of SEQ ID NO: 70; CDR1 comprising the amino acid sequence of SEQ ID NO: 78, CDR2 comprising the amino acid sequence of SEQ ID NO: 79, and CDR3 comprising the amino acid sequence of SEQ ID NO: 80, or an amino acid sequence having at least 80% identity, preferably at least 85% identity, more preferably 90% or 95% identity to the amino acid sequence of SEQ ID NO: 80; or CDR1 comprising the amino acid sequence of SEQ ID NO: 88, CDR2 comprising the amino acid sequence of SEQ ID NO: 89, and CDR3 comprising the amino acid sequence of SEQ ID NO: 90, or an amino acid sequence having at least 80% identity, preferably at least 85% identity, more preferably 90% or 95% identity to the amino acid sequence of SEQ ID NO: 90.

Still further, in the TCR molecule, the α chain variable region comprises CDR1 comprising the amino acid sequence of SEQ ID NO: 3, CDR2 comprising the amino acid sequence of SEQ ID NO: 4, and CDR3 comprising the amino acid sequence of SEQ ID NO: 5, or an amino acid sequence having at least 80% identity, preferably at least 85% identity, more preferably 90% or 95% identity to the amino acid sequence of SEQ ID NO: 5, the β chain variable region comprises CDR1 comprising the amino acid sequence of SEQ ID NO: 8, CDR2 comprising the amino acid sequence of SEQ ID NO: 9, and CDR3 comprising the amino acid sequence of SEQ ID NO: 10, or an amino acid sequence having at least 80% identity, preferably at least 85% identity, more preferably 90% or 95% identity to the amino acid sequence of SEQ ID NO: 10; the α chain variable region comprises CDR1 comprising the amino acid sequence of SEQ ID NO: 13, CDR2 comprising the amino acid sequence of SEQ ID NO: 14, and CDR3 comprising the amino acid sequence of SEQ ID NO: 15, or an amino acid sequence having at least 80% identity, preferably at least 85% identity, more preferably 90% or 95% identity to the amino acid sequence of SEQ ID NO: 15, the β chain variable region comprises CDR1 comprising the amino acid sequence of SEQ ID NO: 18, CDR2 comprising the amino acid sequence of SEQ ID NO: 19, and CDR3 comprising the amino acid sequence of SEQ ID NO: 20, or an amino acid sequence having at least 80% identity, preferably at least 85% identity, more preferably 90% or 95% identity to the amino acid sequence of SEQ ID NO: 20; the α chain variable region comprises CDR1 comprising the amino acid sequence of SEQ ID NO: 23, CDR2 comprising the amino acid sequence of SEQ ID NO: 24, and CDR3 comprising the amino acid sequence of SEQ ID NO: 25, or an amino acid sequence having at least 80% identity, preferably at least 85% identity, more preferably 90% or 95% identity to the amino acid sequence of SEQ ID NO: 25, the β chain variable region comprises CDR1 comprising the amino acid sequence of SEQ ID NO: 28, CDR2 comprising the amino acid sequence of SEQ ID NO: 29, and CDR3 comprising the amino acid sequence of SEQ ID NO: 30, or an amino acid sequence having at least 80% identity, preferably at least 85% identity, more preferably 90% or 95% identity to the amino acid sequence of SEQ ID NO: 30; the α chain variable region comprises CDR1 comprising the amino acid sequence of SEQ ID NO: 33, CDR2 comprising the amino acid sequence of SEQ ID NO: 34, and CDR3 comprising the amino acid sequence of SEQ ID NO: 35, or an amino acid sequence having at least 80% identity, preferably at least 85% identity, more preferably 90% or 95% identity to the amino acid sequence of SEQ ID NO: 35, the β chain variable region comprises CDR1 comprising the amino acid sequence of SEQ ID NO: 38, CDR2 comprising the amino acid sequence of SEQ ID NO: 39, and CDR3 comprising the amino acid sequence of SEQ ID NO: 40, or an amino acid sequence having at least 80% identity, preferably at least 85% identity, more preferably 90% or 95% identity to the amino acid sequence of SEQ ID NO: 40; the α chain variable region comprises CDR1 comprising the amino acid sequence of SEQ ID NO: 43, CDR2 comprising the amino acid sequence of SEQ ID NO: 44, and CDR3 comprising the amino acid sequence of SEQ ID NO: 45, or an amino acid sequence having at least 80% identity, preferably at least 85% identity, more preferably 90% or 95% identity to the amino acid sequence of SEQ ID NO: 45, the β chain variable region comprises CDR1 comprising the amino acid sequence of SEQ ID NO: 48, CDR2 comprising the amino acid sequence of SEQ ID NO: 49, and CDR3 comprising the amino acid sequence of SEQ ID NO: 50, or an amino acid sequence having at least 80% identity, preferably at least 85% identity, more preferably 90% or 95% identity to the amino acid sequence of SEQ ID NO: 50; the α chain variable region comprises CDR1 comprising the amino acid sequence of SEQ ID NO: 53, CDR2 comprising the amino acid sequence of SEQ ID NO: 54, and CDR3 comprising the amino acid sequence of SEQ ID NO: 55, or an amino acid sequence having at least 80% identity, preferably at least 85% identity, more preferably 90% or 95% identity to the amino acid sequence of SEQ ID NO: 55, the β chain variable region comprises CDR1 comprising the amino acid sequence of SEQ ID NO: 58, CDR2 comprising the amino acid sequence of SEQ ID NO: 59, and CDR3 comprising the amino acid sequence of SEQ ID NO: 60, or an amino acid sequence having at least 80% identity, preferably at least 85% identity, more preferably 90% or 95% identity to the amino acid sequence of SEQ ID NO: 60; the α chain variable region comprises CDR1 comprising the amino acid sequence of SEQ ID NO: 63, CDR2 comprising the amino acid sequence of SEQ ID NO: 64, and CDR3 comprising the amino acid sequence of SEQ ID NO: 65, or an amino acid sequence having at least 80% identity, preferably at least 85% identity, more preferably 90% or 95% identity to the amino acid sequence of SEQ ID NO: 65, the β chain variable region comprises CDR1 comprising the amino acid sequence of SEQ ID NO: 68, CDR2 comprising the amino acid sequence of SEQ ID NO: 69, and CDR3 comprising the amino acid sequence of SEQ ID NO: 70, or an amino acid sequence having at least 80% identity, preferably at least 85% identity, more preferably 90% or 95% identity to the amino acid sequence of SEQ ID NO: 70; the α chain variable region comprises CDR1 comprising the amino acid sequence of SEQ ID NO: 73, CDR2 comprising the amino acid sequence of SEQ ID NO: 74, and CDR3 comprising the amino acid sequence of SEQ ID NO: 75, or an amino acid sequence having at least 80% identity, preferably at least 85% identity, more preferably 90% or 95% identity to the amino acid sequence of SEQ ID NO: 75, the β chain variable region comprises CDR1 comprising the amino acid sequence of SEQ ID NO: 78, CDR2 comprising the amino acid sequence of SEQ ID NO: 79, and CDR3 comprising the amino acid sequence of SEQ ID NO: 80, or an amino acid sequence having at least 80% identity, preferably at least 85% identity, more preferably 90% or 95% identity to the amino acid sequence of SEQ ID NO: 80; or the α chain variable region comprises CDR1 comprising the amino acid sequence of SEQ ID NO: 83, CDR2 comprising the amino acid sequence of SEQ ID NO: 84, and CDR3 comprising the amino acid sequence of SEQ ID NO: 85, or an amino acid sequence having at least 80% identity, preferably at least 85% identity, more preferably 90% or 95% identity to the amino acid sequence of SEQ ID NO: 85, the β chain variable region comprises CDR1 comprising the amino acid sequence of SEQ ID NO: 88, CDR2 comprising the amino acid sequence of SEQ ID NO: 89, and CDR3 comprising the amino acid sequence of SEQ ID NO: 90, or an amino acid sequence having at least 80% identity, preferably at least 85% identity, more preferably 90% or 95% identity to the amino acid sequence of SEQ ID NO: 90.

In some specific embodiments, in the TCR molecule, the α chain comprises the amino acid sequence of SEQ ID NO: 1, or an amino acid sequence having at least 80% identity, preferably at least 85% identity, more preferably 90% or 95% identity to the amino acid sequence of SEQ ID NO: 1, and the β chain comprises the amino acid sequence of SEQ ID NO: 6, or an amino acid sequence having at least 80% identity, preferably at least 85% identity, more preferably 90% or 95% identity to the amino acid sequence of SEQ ID NO: 6; the α chain comprises the amino acid sequence of SEQ ID NO: 11 or an amino acid sequence having at least 80% identity, preferably at least 85% identity, more preferably 90% or 95% identity to the amino acid sequence of SEQ ID NO: 11, and the β chain comprises the amino acid sequence of SEQ ID NO: 16 or an amino acid sequence having at least 80% identity, preferably at least 85% identity, more preferably 90% or 95% identity to the amino acid sequence of SEQ ID NO: 16; the α chain comprises the amino acid sequence of SEQ ID NO: 21 or an amino acid sequence having at least 80% identity, preferably at least 85% identity, more preferably 90% or 95% identity to the amino acid sequence of SEQ ID NO: 21, and the β chain comprises the amino acid sequence of SEQ ID NO: 26 or an amino acid sequence having at least 80% identity, preferably at least 85% identity, more preferably 90% or 95% identity to the amino acid sequence of SEQ ID NO: 26; the α chain comprises the amino acid sequence of SEQ ID NO: 31 or an amino acid sequence having at least 80% identity, preferably at least 85% identity, more preferably 90% or 95% identity to the amino acid sequence of SEQ ID NO: 31, and the β chain comprises the amino acid sequence of SEQ ID NO: 36 or an amino acid sequence having at least 80% identity, preferably at least 85% identity, more preferably 90% or 95% identity to the amino acid sequence of SEQ ID NO: 36; the α chain comprises the amino acid sequence of SEQ ID NO: 41 or an amino acid sequence having at least 80% identity, preferably at least 85% identity, more preferably 90% or 95% identity to the amino acid sequence of SEQ ID NO: 41, and the β chain comprises the amino acid sequence of SEQ ID NO: 46 or an amino acid sequence having at least 80% identity, preferably at least 85% identity, more preferably 90% or 95% identity to the amino acid sequence of SEQ ID NO: 46; the α chain comprises the amino acid sequence of SEQ ID NO: 51 or an amino acid sequence having at least 80% identity, preferably at least 85% identity, more preferably 90% or 95% identity to the amino acid sequence of SEQ ID NO: 51, and the β chain comprises the amino acid sequence of SEQ ID NO: 56 or an amino acid sequence having at least 80% identity, preferably at least 85% identity, more preferably 90% or 95% identity to the amino acid sequence of SEQ ID NO: 56; the α chain comprises the amino acid sequence of SEQ ID NO: 61 or an amino acid sequence having at least 80% identity, preferably at least 85% identity, more preferably 90% or 95% identity to the amino acid sequence of SEQ ID NO: 61, and the β chain comprises the amino acid sequence of SEQ ID NO: 66 or an amino acid sequence having at least 80% identity, preferably at least 85% identity, more preferably 90% or 95% identity to the amino acid sequence of SEQ ID NO: 66; the α chain comprises the amino acid sequence of SEQ ID NO: 71 or an amino acid sequence having at least 80% identity, preferably at least 85% identity, more preferably 90% or 95% identity to the amino acid sequence of SEQ ID NO: 71, and the β chain comprises the amino acid sequence of SEQ ID NO: 76 or an amino acid sequence having at least 80% identity, preferably at least 85% identity, more preferably 90% or 95% identity to the amino acid sequence of SEQ ID NO: 76; or the α chain comprises the amino acid sequence of SEQ ID NO: 81 or an amino acid sequence having at least 80% identity, preferably at least 85% identity, more preferably 90% or 95% identity to the amino acid sequence of SEQ ID NO: 81, and the β chain comprises the amino acid sequence of SEQ ID NO: 86 or an amino acid sequence having at least 80% identity, preferably at least 85% identity, more preferably 90% or 95% identity to the amino acid sequence of SEQ ID NO: 86.

Those skilled in the art can understand that, based on the specific sequences provided herein, corresponding variants of the TCR molecules provided herein can be obtained by substituting, deleting, or adding a small number of amino acids and verifying or screening the binding capability or biological activity of the resulting product with the corresponding pMHC complex, and these variants should also be included within the scope of the present invention. For example, the TCR molecules provided herein may have at least one and no more than 10, such as no more than 5, 4, 3, 2, or 1 amino acid alteration(s) in their full-length or variable region sequences or CDR sequences. For example, there may be at least one and no more than 10, such as no more than 5, 4, 3, 2, or 1 amino acid alteration(s) in the α or β variable region sequences, and there may also be a total of no more than 5, 4, 3, 2, or 1 amino acid alteration(s) in the CDR sequences of the α or β variable regions.

It is contemplated that the TCR (or an antigen-specific fragment thereof) described herein may comprise conservative amino acid substitutions. Conservative amino acid substitutions can generally be described as the substitution of one amino acid residue with another amino acid residue having a similar chemical structure, with little or essentially no effect on the function, activity, or other biological properties of the polypeptide. Conservative amino acid substitutions are well known in the art. Conservative substitutions may be, for example, substitution of one amino acid in the following groups (a)-(e) with another amino acid in the same group: (a) small aliphatic nonpolar or weakly polar residues: Ala, Ser, Thr, Pro, and Gly; (b) polar negatively charged residues and their (uncharged) amides: Asp, Asn, Glu, and Gln; (c) polar positively charged residues: His, Arg, and Lys; (d) large aliphatic nonpolar residues: Met, Leu, Ile, Val, and Cys; and (e) aromatic residues: Phe, Tyr, and Trp.

In some embodiments, the TCR (or an antigen-specific fragment thereof) provided herein may further comprise post-translational modifications. Examples of post-translational protein modifications include: phosphorylation, acetylation, methylation, ADP-ribosylation, ubiquitination, glycosylation, carbonylation, sumoylation, biotinylation, or addition of polypeptide side chains or hydrophobic groups. Accordingly, the modified TCR (or an antigen-specific fragment thereof) may comprise non-amino acid components, such as lipids, polysaccharides or monosaccharides, and phosphates. One form of glycosylation is, for example, sialylation modification, which conjugates one or more sialic acid groups to the polypeptide. Sialic acid groups improve the solubility and serum half-life of proteins while also reducing potential immunogenicity of the proteins.

When the N-terminus of the amino acid sequence of the TCR provided herein is a methionine residue (M), as is well known to those skilled in the art, the methionine may be removed during recombinant protein production.

### Fusion Proteins and Immunoconjugates

The present disclosure further provides fusion proteins comprising the variable regions of the α chain and/or β chain of the TCR molecules provided herein, particularly the CDR sequences thereof.

In some embodiments, the fusion protein is in a single-chain TCR form. For example, a complete binding domain having antigen specificity for the corresponding pMHC complex may be formed by linking the α chain variable region and the β chain variable region via a linker sequence.

In some embodiments, the variable regions of the α chain and/or β chain of the TCR molecules provided herein, particularly the CDR sequences thereof, may be utilized to form TCR molecules with multispecificity. In some embodiments, the fusion protein comprises at least two functional moieties, wherein a first functional moiety has antigen specificity for a first pMHC complex, and a second functional moiety has antigen specificity identical to or different from that of the first functional moiety.

In some embodiments, the variable regions of the α chain and/or β chain of the TCR molecules provided herein, particularly the CDR sequences thereof, may be utilized to form fusion proteins with multispecificity. In some embodiments, the fusion protein comprises at least two functional moieties, wherein a first functional moiety has antigen specificity for a pMHC complex, and a second functional moiety has antigen specificity or targeting capability different from that of the first functional moiety. In some embodiments, the second functional moiety may be a targeting moiety capable of specifically recognizing and binding to tumor-specific antigens, tumor-associated antigens, or other target cell surface molecules, for example, an antibody or an antigen-binding fragment thereof (e.g., scFv), a hormone, a growth factor, a cytokine, and any other natural or unnatural ligand that binds to a cell surface receptor (e.g., epidermal growth factor receptor (EGFR), CD28, platelet-derived growth factor receptor (PDGFR), nicotinic acetylcholine receptor (nAChR), etc.).

In some embodiments, the binding domain of an antibody molecule (e.g., a classical tetrameric antibody molecule) may be replaced with the TCR molecule (or the α and β chain variable regions or the binding domain formed thereby) provided herein to form a recombinant antibody molecule having the antigen specificity of the TCR molecule. In some embodiments, the α chain variable region of the TCR molecule replaces the two heavy chain variable regions of the antibody molecule linked to the heavy chain constant region, while the β chain variable region replaces the two light chain variable regions of the antibody molecule linked to the light chain constant region. In some embodiments, the β chain variable region of the TCR molecule replaces the two heavy chain variable regions of the antibody molecule linked to the heavy chain constant region, while the α chain variable region replaces the two light chain variable regions of the antibody molecule linked to the light chain constant region. In some embodiments, the α chain variable region and the β chain variable region of the TCR molecule replace one heavy chain variable region and one light chain variable region of the antibody molecule, respectively, while retaining an original antigen-binding domain of the antibody molecule unchanged, thereby forming a bispecific recombinant antibody molecule targeting the pMHC complex and the target antigen of the original antibody molecule, respectively. The recombinant antibody molecule thus formed may achieve effector functions of the antibody molecule via its constant region (e.g., Fc fragment), such as mediating complement-dependent cytotoxicity (CDC), antibody-dependent cell-mediated cytotoxicity (ADCC), mediating phagocytosis, etc. Additionally, fusion with the Fc fragment may increase the *in vivo* half-life of the TCR molecule (or the α and β chain variable regions or the binding domain formed thereof), thereby increasing the dosing interval when the TCR molecule (or the α and β chain variable regions or the binding domain formed thereof) is used as a therapeutic drug. In some embodiments, the Fc fragment may be derived from a constant region of an immunoglobulin, such as IgG1, IgG2, or IgG4.

In some embodiments, the TCR molecule (or the α and β chain variable regions or the binding domain formed thereof) provided herein may be used as an extracellular binding domain to construct a chimeric antigen receptor (CAR). For example, the α and β chain variable regions may be linked via a linker sequence to form a binding molecule similar to an scFv, replacing the extracellular binding domain of a conventional CAR molecule and connecting it to the transmembrane region and intracellular signaling domain. This CAR construct targets the pMHC complex corresponding to the original TCR molecule and activates the T cells expressing the CAR through the intracellular signaling domain of the CAR molecule to achieve desired immunological effects (e.g., target cell killing function).

In some embodiments, the TCR molecule (or the α and β chain variable regions or the binding domain formed thereof) may be conjugated to a conjugate to form an immunoconjugate, wherein the conjugate is, for example, a detectable label, a therapeutic agent, a tracer, etc.

In some embodiments, the TCR molecule (or the α and β chain variable regions or the binding domain formed thereof) may be conjugated to a protein tag to form a fusion protein. Protein tags may include purification tags and detectable tags. Purification tags include, but are not limited to, His6 tag, Flag tag, MBP tag, GST tag, SUMO tag, etc. Detectable tags may be used to indicate the presence or quantity of the TCR molecule (or the α and β chain variable regions or the binding domain formed thereof) in a sample, or to track the location of the TCR molecule (or the α and β chain variable regions or the binding domain formed thereof) in a subject or within cells. Examples of detectable tags include various enzymes usable in immunoassays, such as horseradish peroxidase (HRP), alkaline phosphatase (ALP), etc.; and fluorescent proteins, such as GFP. Due to the specific binding capability of the TCR molecule (or the α and β chain variable regions or the binding domain formed thereof) to the corresponding pMHC complex, the quantity of the TCR molecule (or the α and β chain variable regions or the binding domain formed thereof) may be determined by the amount of the detectable tag conjugated thereto, and further the content of the corresponding pMHC complex in the sample may be determined. When the corresponding pMHC complex serves as a tumor marker, the fusion protein may be used for tumor detection and diagnosis.

In other embodiments, the TCR molecule (or the α and β chain variable regions or the binding domain formed thereof) provided herein may be linked to a cytokine or therapeutic protein to form a fusion protein. In this case, the cytokine or therapeutic protein may be purposefully delivered to specific tissues or cells by leveraging the specific binding capability of the TCR molecule (or the α and β chain variable regions or the binding domain formed thereof) to the corresponding pMHC complex, thereby achieving the therapeutic effect of the cytokine or therapeutic protein.

In some embodiments, the TCR molecule (or the α and β chain variable regions or the binding domain formed thereof) may be linked to a chemotherapeutic agent, for example, asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc., so as to deliver the chemotherapeutic agent to specific cells or tissues via the antigen specificity of the TCR molecule.

In some embodiments, the TCR molecule (or the α and β chain variable regions or the binding domain formed thereof) may be conjugated to a radioisotope (e.g., ³H, ¹⁴C, ³⁵S) for tracing or therapeutic purposes.

### Host Cells Expressing TCR Molecules

The present disclosure further provides host cells expressing the TCR molecule (or the α and β chain variable regions or the binding domain formed thereof) provided herein.

The host cells used refer to any type of cell capable of expressing the TCR molecule (or the α and β chain variable regions or the binding domain formed thereof) provided herein. Preferably, the host cell expresses a complete TCR molecule, namely comprising the α and β chain variable regions, α and β chain constant regions, transmembrane region, and intracellular region. The host cell may be a eukaryotic cell or a prokaryotic cell. Preferably, the host cell is preferably a mammalian cell. Most preferably, the host cell is a human cell. Although the host cell may be a cell of any cell type, derived from any type of tissue, and may be at any developmental stage, the host cell is preferably a peripheral blood lymphocyte (PBL) or peripheral blood mononuclear cell (PBMC). More preferably, the host cell is a T cell. For the purposes herein, the T cell may be any T cell, such as a cultured T cell, e.g., a primary T cell or a T cell from a cultured T cell line such as Jurkat, SupT1, etc., or a T cell obtained from a mammal. If obtained from a mammal, the T cell may be obtained from many sources including, but not limited to, blood, bone marrow, lymph nodes, thymus, or other tissues or fluids. The T cell may also be enriched or purified. The T cell may be any type of T cell and may be at any developmental stage, including but not limited to: CD4+/CD8+ double-positive T cells, CD4+ helper T cells such as Th1 and Th2 cells, CD4+ T cells, CD8+ T cells (e.g., cytotoxic T cells), tumor-infiltrating lymphocytes (TIL), memory T cells (e.g., central memory T cells and effector memory T cells), naive T cells, etc.

The present disclosure further provides host cells expressing the CAR provided herein. Preferably, the host cell is a human cell, particularly a human T cell or NK cell.

The effector function of the host cells (e.g., T cells) expressing the TCR or CAR may be detected in various ways. In some embodiments, the killing capability of the host cells against target cells is reflected by incubating host cells (e.g., T cells) expressing the TCR or CAR with target cells and detecting the quantity or activity of the target cells (e.g., by detecting LDH release). In some embodiments, the immune effector function of the host cells is assessed by incubating host cells (e.g., T cells) expressing the TCR or CAR with target cells and detecting IFN-y secretion or CD69 expression in the host cells.

### Pharmaceutical Compositions and Treatment Methods

The TCR molecule (or the α and β chain variable regions or the binding domain formed thereof) provided herein, and fusion proteins or immunoconjugates comprising the TCR molecule (or the α and β chain variable regions or the binding domain formed thereof) may be formulated with a pharmaceutically acceptable carrier into a pharmaceutical composition for administration to a subject for tumor prevention or treatment.

In some implementations, the TCR molecule (or the α and β chain variable regions or the binding domain formed thereof) provided herein, and fusion proteins or immunoconjugates comprising the TCR molecule (or the α and β chain variable regions or the binding domain formed thereof) may be administered in combination with one or more other drugs (e.g., anti-tumor agents).

In some embodiments, the disease or disorder being treated is a tumor, particularly a tumor expressing an antigen peptide VVGAVGVGK (SEQ ID NO: 91) or VVVGAVGVGK (SEQ ID NO: 92). These tumors include, but are not limited to, acute lymphoblastic cancer, acute myeloid leukemia, alveolar rhabdomyosarcoma, bone cancer, brain cancer, breast cancer, anal cancer, anal canal cancer or anorectal cancer, eye cancer, intrahepatic bile duct cancer, joint cancer, cervical cancer, gallbladder cancer or pleural cancer, nasal cancer, nasal cavity cancer or middle ear cancer, oral cancer, vaginal cancer, vulvar cancer, chronic lymphocytic leukemia, chronic myeloid cancer, colon cancer, colorectal cancer, endometrial cancer, esophageal cancer, cervical cancer, gastrointestinal carcinoid tumor, glioma, Hodgkin lymphoma, hypopharyngeal cancer, kidney cancer, laryngeal cancer, liver cancer, lung cancer, malignant mesothelioma, melanoma, multiple myeloma, nasopharyngeal cancer, non-Hodgkin lymphoma, oropharyngeal cancer, ovarian cancer, penile cancer, pancreatic cancer, peritoneal cancer, omental cancer and mesenteric cancer, pharyngeal cancer, prostate cancer, rectal cancer, renal cancer, skin cancer, small intestine cancer, soft tissue cancer, stomach cancer, testicular cancer, thyroid cancer, uterine cancer, ureteral cancer, and bladder cancer. Preferred cancers are pancreatic cancer, colorectal cancer, lung cancer, endometrial cancer, ovarian cancer, prostate cancer, etc.

Nucleic acid molecules encoding the TCR molecule (or the α and β chain variable regions or the binding domain formed thereof) provided herein, and fusion proteins or immunoconjugates comprising the TCR molecule (or the α and β chain variable regions or the binding domain formed thereof), expression vectors comprising the nucleic acid molecules, and host cells (T cells, CAR cells) transfected with the nucleic acid molecules or expression vectors may also be used for the above therapeutic purposes in various ways. For example, for expression vectors, they may be introduced into a subject via gene therapy means known in the art to express the target protein or polypeptide (e.g., the TCR molecule (or the α and β chain variable regions or the binding domain formed thereof) provided herein, and fusion proteins or immunoconjugates comprising the TCR molecule (or the α and β chain variable regions or the binding domain formed thereof)), thereby achieving therapeutic purposes.

The effective amount for this use may depend on the severity of the disease and the overall state of the patient's own immune system. The dosing regimen will also vary with the disease state and the subject's condition, and generally may range from a single bolus administration or continuous infusion to multiple administrations per day (e.g., every 4-6 hours). A skilled clinician can readily determine whether a subject is a candidate for such treatment, for example, by utilizing clinical trials, physical examination, and the subject's family history.

### Detection or Treatment Kits

The TCR molecule (or the α and β chain variable regions or the binding domain formed thereof) provided herein, and fusion proteins or immunoconjugates comprising the TCR molecule (or the α and β chain variable regions or the binding domain formed thereof) may specifically bind to the corresponding pMHC complex in a sample. By detecting the amount of the formed ternary complex (i.e., TCR-antigen peptide-MHC molecule), the content (or presence) of the corresponding pMHC complex in the sample may be conveniently determined.

As described above, for this purpose, the TCR molecule (or the α and β chain variable regions or the binding domain formed thereof) provided herein may be conjugated to various detection labels, facilitating detection by various means, including but not limited to bioluminescence, fluorescence, radiolabeling, product yield from enzymatic reactions, etc. After detecting the content of the corresponding pMHC complex in the sample and comparing it with the normal content in a normal population, the disease status or severity of the subject providing the sample may be determined. By repeatedly detecting changes in the content of the corresponding pMHC complex over time during the subject's treatment, the efficacy of the treatment method may also be determined, thereof providing a basis for modifying the treatment regimen.

The TCR molecule (or the α and β chain variable regions or the binding domain formed thereof) provided herein, and fusion proteins or immunoconjugates comprising the TCR molecule (or the α and β chain variable regions or the binding domain formed thereof), or host cells may be placed in containers to form detection or treatment kits. The containers may be in the form of boxes, ampoules, vials, tubes, bags, or other suitable containers known in the art. The containers may be made of plastic, glass, laminated paper, metal foil, or other materials suitable for preserving pharmaceuticals. If needed, instructions for use are also provided with the container. The instructions typically may include information on how to use the TCR molecule (or the α and β chain variable regions or the binding domain formed thereof), compositions comprising the TCR molecule (or the α and β chain variable regions or the binding domain formed thereof), or host cells for treating or preventing tumors, and may include, for example, a description of the therapeutic agent (e.g., TCR molecule (or the α and β chain variable regions or the binding domain formed thereof), nucleic acid molecules, host cells, etc.); dosing regimens for treating or preventing neoplasia; precautions; warnings; indications; contraindications; adverse reactions; animal pharmacology; clinical studies; and/or references. The instructions may be printed directly on the container (if present), or as a label affixed to the container, or as a separate sheet, booklet, card, or folded insert provided in or with the container.

### Experimental materials and reagents:

1. Healthy human PBMCs
2. Targeting peptides:
   A*11:01 KRAS-G12V-9 peptide: VVGAVGVGK (SEQ ID NO: 91)
   A*11:01 KRAS-G12V-10 peptide: VVVGAVGVGK (SEQ ID NO: 92)
3. Brilliant Violet 510^{™} anti-human CD3 Antibody: Biolegend 300448
4. APC anti-human CD8a Antibody: Biolegend 300912
5. PE-KRAS-G12V-Pentamer: PROIMMUNE
6. Chromium Next GEM Single Cell V(D)J Reagent Kits v1.1
7. Lentiviral vectors
8. Polybrene viral enhancers: merck millipore TR-1003-G
9. Anti-CD3 /CD28 magnetic beads: novoprotein GMP-B038
10. X-VIVO 15:lonza 04418q
11. OKM-100: CansBio sj18
12. RPMI 1640: gibco 11875-093
13. LDH kit: dojindo
14. Human IFN- γ kit: Excell Bio EH008-96
15. IFN- γ ELISPOT: Mabtech 3420-4AST-10
16. APC-anti-human CD69 Antibody: biolegend 310910

### Instruments and equipments

Flow cytometer: BD LSRFortessa
Flow sorter: SONY SH800S
Microplate reader: Molecular Devices SpectraMax i3x
ELISPOT imager: Bio-sys Bioreader BIO-SYS Bioreader 6000-Eβ

### Graphing and analysis softwares

FlowJo
GraphPad Prism 8.0.1

### Example

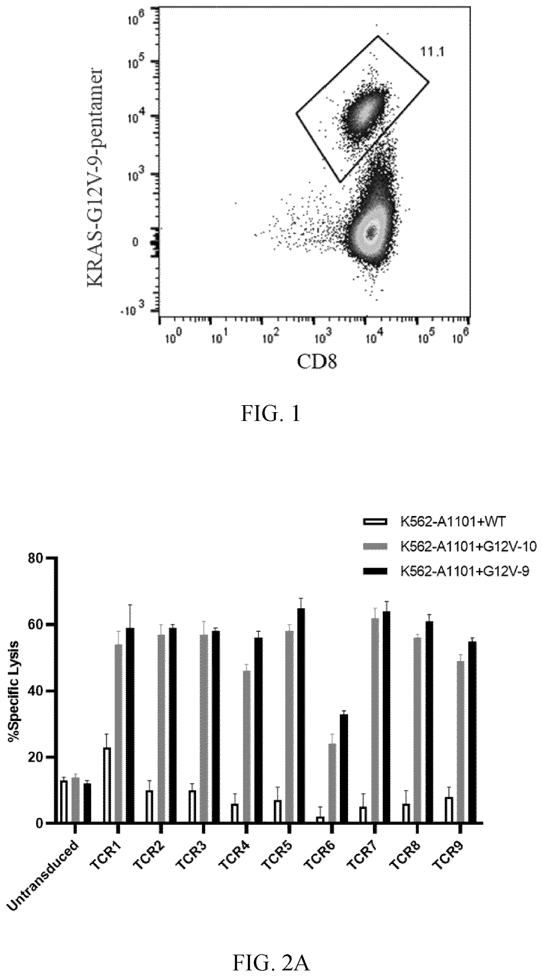

T cells from three healthy donors were stimulated with dendritic cells loaded with KRAS G12V 9 peptide VVGAVGVGK (SEQ ID NO: 91), after which the proportion of A*11:01-G12V-specific T cells was detected by pentamer staining and flow cytometer BD LSRFortessa. Subsequently, the specific T cells were enriched and sorted via SONY SH800S flow sorter and analyzed using FlowJo software. The analyzed flow cytometry plots are shown in Figure 1. The enriched cells were subjected to 10× single-cell sequencing to obtain paired TCRα chain and TCRβ chain.

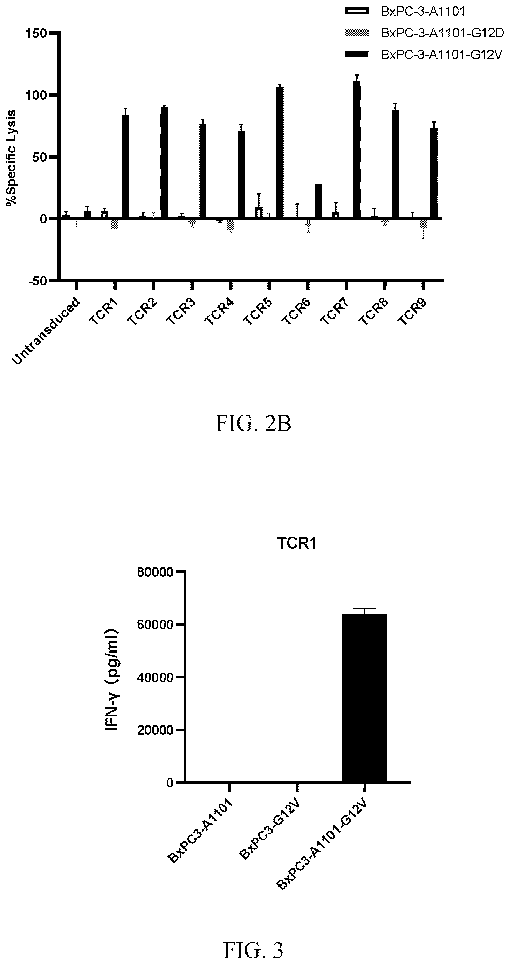

A lentiviral vector containing the TCR gene of the present invention was constructed and used to transduce T cells. The T cells transduced with different TCRs were co-cultured with: (1) HLA-A*11:01-expressing K562 cells loaded with different peptides (G12V-9 peptide: VVGAVGVGK (SEQ ID NO: 91), G12V-10 peptide: VVVGAVGVGK (SEQ ID NO: 92), or WT peptide: VVGAGGVGK (SEQ ID NO: 93) + VVVGAGGVGK (SEQ ID NO: 94)); and (2) BxPC3 cells expressing HLA-A*11:01, co-expressing HLA-A*11:01 and KRAS G12V, or co-expressing HLA-A*11:01 and KRAS G12D (effector cell: target cell = 5:1). After co-incubation for 16 h, the killing ability of T cells against target cells was evaluated by detecting LDH release. Statistical data are shown in Figures 2A and 2B (GraphPad Prism 8.0.1 software). The T cells transduced with different TCRs exhibited effective specific killing against target cells expressing A*11:01 loaded with KRAS G12V-9 peptide or KRAS G12V-10 peptides, as well as those overexpressing KRAS G12V.

IFN-γ release by T cells transduced with specified TCRs stimulated with KRAS G12V-9 peptide (VVGAVGVGK) (SEQ ID NO: 91) was detected (effector: target = 1: 1). As shown in Figure 3, the primary T cells expressing exemplary TCRs of the present disclosure produced substantial IFN-γ only upon target stimulation.

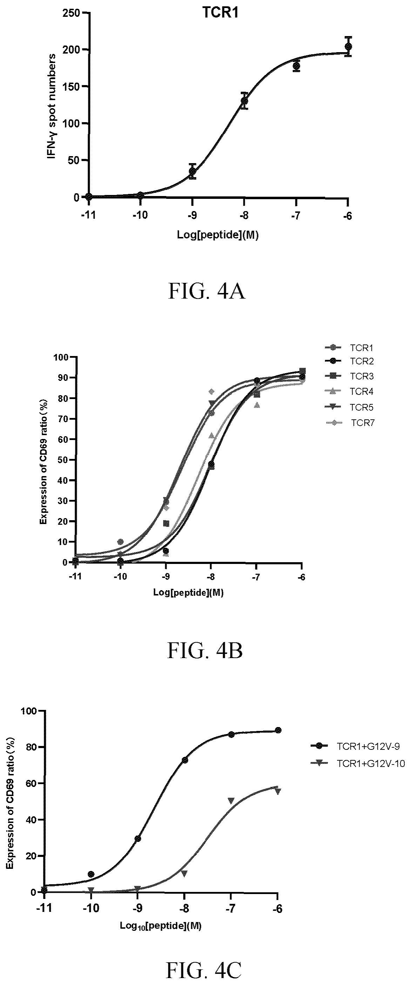

EC50 values of different TCRs were assessed by spot numbers of IFN-γ release and CD69 expression after co-culture of specific TCR-transduced T cells with target cells loaded with graded concentrations of KRAS G12V-9 peptide (VVGAVGVGK) (SEQ ID NO: 91). As shown in Figures 4A and 4B, EC50 concentrations of TCRs recognizing KRAS G12V-9 peptide range from 1 nM-10 nM. As shown in Figure 4C, EC50 concentrations of TCR1 recognizing KRAS G12V-9 peptide and the 10-peptide peptide differed by approximately 10-fold (9-mer peptide: 2.24 nM; 10-mer peptide: 31.3 nM).

The partial amino acid sequences mentioned in this disclosure are as follows.
TCR1 α chain amino acid sequence 
MRQVARVIVFLTLSTLSLAKTTQPISMDSYEGQEVNITCSHNNIAT
NDYITWYQQFPSQGPRFIIQGYKTKVTNEVASLFIPADRKSSTLSLPRVS
LSDTAVYYCLVGALSSSWYGQNFVFGPGTRLSVLPYIQNPDPAVYQLR
DSKSSDKSVCLFTDFDSQTNVSQSKDSDVYITDKTVLDMRSMDFKSNS
AVAWSNKSDFACANAFNNSIIPEDTFFPSPESSCDVKLVEKSFETDTNLN
FQNLSVIGFRILLLKVAGFNLLMTLRLWSS (SEQ ID NO: 1)
TCR1 α chain V region amino acid sequence 
LAKTTQPISMDSYEGQEVNITCSHNNIATNDYITWYQQFPSQGPRFI
IQGYKTKVTNEVASLFIPADRKSSTLSLPRVSLSDTAVYYCLVGALSSS
WYGQNFVFGPGTRLSVLP (SEQ ID NO: 2)
TCR1 α chain CDR1 amino acid sequence
   NIATNDY (SEQ ID NO: 3)
TCR1 α chain CDR2 amino acid sequence
   GYKTK (SEQ ID NO: 4)
TCR1 α chain CDR3 amino acid sequence
   LVGALSSSWYGQNFV (SEQ ID NO: 5)
TCR1 β chain amino acid sequence 
MGCRLLCCVVFCLLQAGPLDTAVSQTPKYLVTQMGNDKSIKCEQ
NLGHDTMYWYKQDSKKFLKIMFSYNNKELIINETVPNRFSPKSPDKAH
LNLHINSLELGDSAVYFCASSQSGAQDGYTFGSGTRLTVVEDLNKVFPP
EVAVFEPSEAEISHTQKATLVCLATGFFPDHVELSWWVNGKEVHSGVS
TDPQPLKEQPALNDSRYCLSSRLRVSATFWQNPRNHFRCQVQFYGLSE
NDEWTQDRAKPVTQIVSAEAWGRADCGFTSVSYQQGVLSATILYEILL
GKATLYAVLVSALVLMAMVKRKDF (SEQ ID NO: 6)
TCR1 β chain V region amino acid sequence 
DTAVSQTPKYLVTQMGNDKSIKCEQNLGHDTMYWYKQDSKKFL
KIMFSYNNKELIINETVPNRFSPKSPDKAHLNLHINSLELGDSAVYFCAS
SQSGAQDGYTFGSGTRLTVV (SEQ ID NO: 7)
TCR1 β chain CDR1 amino acid sequence
   LGHDT (SEQ ID NO: 8)
TCR1 β chain CDR2 amino acid sequence
   YNNKEL (SEQ ID NO: 9)
TCR1 β chain CDR1 amino acid sequence
   ASSQSGAQDGYT (SEQ ID NO: 10)
TCR2 α chain amino acid sequence 
MAMLLGASVLILWLQPDWVNSQQKNDDQQVKQNSPSLSVQEGRI
SILNCDYTNSMFDYFLWYKKYPAEGPTFLISISSIKDKNEDGRFTVFLNK
SAKHLSLHIVPSQPGDSAVYFCAAKDNAGNMLTFGGGTRLMVKPHIQN
PDPAVYQLRDSKSSDKSVCLFTDFDSQTNVSQSKDSDVYITDKTVLDM
RSMDFKSNSAVAWSNKSDFACANAFNNSIIPEDTFFPSPESSCDVKLVE
KSFETDTNLNFQNLSVIGFRILLLKVAGFNLLMTLRLWSS (SEQ ID NO:
11)
TCR2 α chain V region amino acid sequence 
DQQVKQNSPSLSVQEGRISILNCDYTNSMFDYFLWYKKYPAEGPT
FLISISSIKDKNEDGRFTVFLNKSAKHLSLHIVPSQPGDSAVYFCAAKDN
AGNMLTFGGGTRLMVKP (SEQ ID NO: 12)
TCR2 α chain CDR1 amino acid sequence
   NSMFDY (SEQ ID NO: 13)
TCR2 α chain CDR2 amino acid sequence
   ISSIKDK (SEQ ID NO: 14)
TCR2 α chain CDR3 amino acid sequence
   AAKDNAGNMLT (SEQ ID NO: 15)
TCR2 β chain amino acid sequence 
MGSRLLCWVLLCLLGAGPVKAGVTQTPRYLIKTRGQQVTLSCSPI
SGHRSVSWYQQTPGQGLQFLFEYFSETQRNKGNFPGRFSGRQFSNSRSE
MNVSTLELGDSALYLCASSLGTAEAFFGQGTRLTVVEDLNKVFPPEVA
VFEPSEAEISHTQKATLVCLATGFFPDHVELSWWVNGKEVHSGVSTDP
QPLKEQPALNDSRYCLSSRLRVSATFWQNPRNHFRCQVQFYGLSENDE
WTQDRAKPVTQIVSAEAWGRADCGFTSVSYQQGVLSATILYEILLGKA
TLYAVLVSALVLMAMVKRKDF (SEQ ID NO: 16)
TCR2 β chain V region amino acid sequence 
KAGVTQTPRYLIKTRGQQVTLSCSPISGHRSVSWYQQTPGQGLQFL
FEYFSETQRNKGNFPGRFSGRQFSNSRSEMNVSTLELGDSALYLCASSL
GTAEAFFGQGTRLTVV (SEQ ID NO: 17)
TCR2 β chain CDR1 amino acid sequence
   SGHRS (SEQ ID NO: 18)
TCR2 β chain CDR2 amino acid sequence
   YFSETQ (SEQ ID NO: 19)
TCR2 β chain CDR3 amino acid sequence
   ASSLGTAEAF (SEQ ID NO: 20)
TCR3 α chain amino acid sequence 
MAMLLGASVLILWLQPDWVNSQQKNDDQQVKQNSPSLSVQEGRI
SILNCDYTNSMFDYFLWYKKYPAEGPTFLISISSIKDKNEDGRFTVFLNK
SAKHLSLHIVPSQPGDSAVYFCAANSGYALNFGKGTSLLVTPHIQNPDP
AVYQLRDSKSSDKSVCLFTDFDSQTNVSQSKDSDVYITDKTVLDMRSM
DFKSNSAVAWSNKSDFACANAFNNSIIPEDTFFPSPESSCDVKLVEKSFE
TDTNLNFQNLSVIGFRILLLKVAGFNLLMTLRLWSS (SEQ ID NO: 21)
TCR3 α chain V region amino acid sequence 
DQQVKQNSPSLSVQEGRISILNCDYTNSMFDYFLWYKKYPAEGPT
FLISISSIKDKNEDGRFTVFLNKSAKHLSLHIVPSQPGDSAVYFCAANSG
YALNFGKGTSLLVTP (SEQ ID NO: 22)
TCR3 α chain CDR1 amino acid sequence
   NSMFDY (SEQ ID NO: 23)
TCR3 α chain CDR2 amino acid sequence
   ISSIKDK (SEQ ID NO: 24)
TCR3 α chain CDR3 amino acid sequence
   AANSGYALN (SEQ ID NO: 25)
TCR3 β chain amino acid sequence 
MGSRLLCWVLLCLLGAGPVKAGVTQTPRYLIKTRGQQVTLSCSPI
SGHRSVSWYQQTPGQGLQFLFEYFSETQRNKGNFPGRFSGRQFSNSRSE
MNVSTLELGDSALYLCASSLGGVGNEQFFGPGTRLTVLEDLKNVFPPE
VAVFEPSEAEISHTQKATLVCLATGFYPDHVELSWWVNGKEVHSGVST
DPQPLKEQPALNDSRYCLSSRLRVSATFWQNPRNHFRCQVQFYGLSEN
DEWTQDRAKPVTQIVSAEAWGRADCGFTSESYQQGVLSATILYEILLG
KATLYAVLVSALVLMAMVKRKDSRG (SEQ ID NO: 26)
TCR3 β chain V region amino acid sequence 
KAGVTQTPRYLIKTRGQQVTLSCSPISGHRSVSWYQQTPGQGLQFL
FEYFSETQRNKGNFPGRFSGRQFSNSRSEMNVSTLELGDSALYLCASSL
GGVGNEQFFGPGTRLTVL (SEQ ID NO: 27)
TCR3 β chain CDR1 amino acid sequence
   SGHRS (SEQ ID NO: 28)
TCR3 β chain CDR2 amino acid sequence
   YFSETQ (SEQ ID NO: 29)
TCR3 β chain CDR3 amino acid sequence
   ASSLGGVGNEQF (SEQ ID NO: 30)
TCR4 α chain amino acid sequence 
MMKSLRVLLVILWLQLSWVWSQQKEVEQNSGPLSVPEGAIASLN
CTYSDRGSQSFFWYRQYSGKSPELIMFIYSNGDKEDGRFTAQLNKASQ
YVSLLIRDSQPSDSATYLCAVERGSTLGRLYFGRGTQLTVWPDIQNPDP
AVYQLRDSKSSDKSVCLFTDFDSQTNVSQSKDSDVYITDKTVLDMRSM
DFKSNSAVAWSNKSDFACANAFNNSIIPEDTFFPSPESSCDVKLVEKSFE
TDTNLNFQNLSVIGFRILLLKVAGFNLLMTLRLWSS (SEQ ID NO: 31)
TCR4 α chain V region amino acid sequence 
QKEVEQNSGPLSVPEGAIASLNCTYSDRGSQSFFWYRQYSGKSPEL
IMFIYSNGDKEDGRFTAQLNKASQYVSLLIRDSQPSDSATYLCAVERGS
TLGRLYFGRGTQLTVWP (SEQ ID NO: 32)
TCR4 α chain CDR1 amino acid sequence
   DRGSQS (SEQ ID NO: 33)
TCR4 α chain CDR2 amino acid sequence
   IYSNGD (SEQ ID NO: 34)
TCR4 α chain CDR3 amino acid sequence
   AVERGSTLGRLY (SEQ ID NO: 35)
TCR4 β chain amino acid sequence 
MGTSLLCWVVLGFLGTDSVSTDHTGAGVSQSPRYKVTKRGQDVT
LRCDPISSHATLYWYQQALGQGPEFLTYFNYEAQPDKSGLPSDRFSAER
PEGSISTLTIQRTEQRDSAMYRCASSLSSPTGGPINEQFFGPGTRLTVLED
LKNVFPPEVAVFEPSEAEISHTQKATLVCLATGFYPDHVELSWWVNGK
EVHSGVSTDPQPLKEQPALNDSRYCLSSRLRVSATFWQNPRNHFRCQV
QFYGLSENDEWTQDRAKPVTQIVSAEAWGRADCGFTSESYQQGVLSA
TILYEILLGKATLYAVLVSALVLMAMVKRKDSRG (SEQ ID NO: 36)
TCR4 β chain V region amino acid sequence 
GAGVSQSPRYKVTKRGQDVTLRCDPISSHATLYWYQQALGQGPE
FLTYFNYEAQPDKSGLPSDRFSAERPEGSISTLTIQRTEQRDSAMYRCAS
SLSSPTGGPINEQFFGPGTRLTVL (SEQ ID NO: 37)
TCR4 β chain CDR1 amino acid sequence
   SSHAT (SEQ ID NO: 38)
TCR4 β chain CDR2 amino acid sequence
   FNYEAQ (SEQ ID NO: 39)
TCR4 β chain CDR3 amino acid sequence
   ASSLSSPTGGPINEQF (SEQ ID NO: 40)
TCR5 α chain amino acid sequence 
MKTFAGFSFLFLWLQLDCMSRGEDVEQSLFLSVREGDSSVINCTY
TDSSSTYLYWYKQEPGAGLQLLTYIFSNMDMKQDQRLTVLLNKKDKH
LSLRIADTQTGDSAIYFCAESPGGGADGLTFGKGTHLIIQPYIQNPDPAV
YQLRDSKSSDKSVCLFTDFDSQTNVSQSKDSDVYITDKTVLDMRSMDF
KSNSAVAWSNKSDFACANAFNNSIIPEDTFFPSPESSCDVKLVEKSFETD
TNLNFQNLSVIGFRILLLKVAGFNLLMTLRLWSS (SEQ ID NO: 41)
TCR5 α chain V region amino acid sequence 
GEDVEQSLFLSVREGDSSVINCTYTDSSSTYLYWYKQEPGAGLQL
LTYIFSNMDMKQDQRLTVLLNKKDKHLSLRIADTQTGDSAIYFCAESP
GGGADGLTFGKGTHLIIQP (SEQ ID NO: 42)
TCR5 α chain CDR1 amino acid sequence
   DSSSTY (SEQ ID NO: 43)
TCR5 α chain CDR2 amino acid sequence
   IFSNMDM (SEQ ID NO: 44)
TCR5 α chain CDR3 amino acid sequence
   AESPGGGADGLT (SEQ ID NO: 45)
TCR5 β chain amino acid sequence 
MLLLLLLLGPGSGLGAVVSQHPSWVICKSGTSVKIECRSLDFQATT
MFWYRQFPKQSLMLMATSNEGSKATYEQGVEKDKFLINHASLTLSTLT
VTSAHPEDSSFYICSAPRGWAAGVYGYTFGSGTRLTVVEDLNKVFPPE
VAVFEPSEAEISHTQKATLVCLATGFFPDHVELSWWVNGKEVHSGVST
DPQPLKEQPALNDSRYCLSSRLRVSATFWQNPRNHFRCQVQFYGLSEN
DEWTQDRAKPVTQIVSAEAWGRADCGFTSVSYQQGVLSATILYEILLG
KATLYAVLVSALVLMAMVKRKDF (SEQ ID NO: 46)
TCR5 β chain V region amino acid sequence 
GAVVSQHPSWVICKSGTSVKIECRSLDFQATTMFWYRQFPKQSLM
LMATSNEGSKATYEQGVEKDKFLINHASLTLSTLTVTSAHPEDSSFYICS
APRGWAAGVYGYTFGSGTRLTVV (SEQ ID NO: 47)
TCR5 β chain CDR1 amino acid sequence
   DFQATT (SEQ ID NO: 48)
TCR5 β chain CDR2 amino acid sequence
   SNEGSKA (SEQ ID NO: 49)
TCR5 β chain CDR3 amino acid sequence
   SAPRGWAAGVYGYT (SEQ ID NO: 50)
TCR6 α chain amino acid sequence 
MLLELIPLLGIHFVLRTARAQSVTQPDIHITVSEGASLELRCNYSYG
ATPYLFWYVQSPGQGLQLLLKYFSGDTLVQGIKGFEAEFKRSQSSFNLR
KPSVHWSDAAEYFCAVGASREYGNKLVFGAGTILRVKSYIQNPDPAVY
QLRDSKSSDKSVCLFTDFDSQTNVSQSKDSDVYITDKTVLDMRSMDFK
SNSAVAWSNKSDFACANAFNNSIIPEDTFFPSPESSCDVKLVEKSFETDT
NLNFQNLSVIGFRILLLKVAGFNLLMTLRLWSS (SEQ ID NO: 51)
TCR6 α chain V region amino acid sequence 
AQSVTQPDIHITVSEGASLELRCNYSYGATPYLFWYVQSPGQGLQL
LLKYFSGDTLVQGIKGFEAEFKRSQSSFNLRKPSVHWSDAAEYFCAVG
ASREYGNKLVFGAGTILRVKS (SEQ ID NO: 52)
TCR6 α chain CDR1 amino acid sequence
   YGATPY (SEQ ID NO: 53)
TCR6 α chain CDR2 amino acid sequence
   YFSGDTLV (SEQ ID NO: 54)
TCR6 α chain CDR3 amino acid sequence
   AVGASREYGNKLV (SEQ ID NO: 55)
TCR6 β chain amino acid sequence 
MDTRVLCCAVICLLGAGLSNAGVMQNPRHLVRRRGQEARLRCSP
MKGHSHVYWYRQLPEEGLKFMVYLQKENIIDESGMPKERFSAEFPKEG
PSILRIQQVVRGDSAAYFCASSPGGLLHFGNGTRLTVTEDLNKVFPPEV
AVFEPSEAEISHTQKATLVCLATGFFPDHVELSWWVNGKEVHSGVSTD
PQPLKEQPALNDSRYCLSSRLRVSATFWQNPRNHFRCQVQFYGLSEND
EWTQDRAKPVTQIVSAEAWGRADCGFTSVSYQQGVLSATILYEILLGK
ATLYAVLVSALVLMAMVKRKDF (SEQ ID NO: 56)
TCR6 β chain V region amino acid sequence 
NAGVMQNPRHLVRRRGQEARLRCSPMKGHSHVYWYRQLPEEGL
KFMVYLQKENIIDESGMPKERFSAEFPKEGPSILRIQQVVRGDSAAYFC
ASSPGGLLHFGNGTRLTVT (SEQ ID NO: 57)
TCR6 β chain CDR1 amino acid sequence
   KGHSH (SEQ ID NO: 58)
TCR6 β chain CDR2 amino acid sequence
   LQKENI (SEQ ID NO: 59)
TCR6 β chain CDR3 amino acid sequence
   ASSPGGLLH (SEQ ID NO: 60)
TCR7 α chain amino acid sequence 
MAMLLGASVLILWLQPDWVNSQQKNDDQQVKQNSPSLSVQEGRI
SILNCDYTNSMFDYFLWYKKYPAEGPTFLISISSIKDKNEDGRFTVFLNK
SAKHLSLHIVPSQPGDSAVYFCAGKTNTGNQFYFGTGTSLTVIPNIQNP
DPAVYQLRDSKSSDKSVCLFTDFDSQTNVSQSKDSDVYITDKTVLDMR
SMDFKSNSAVAWSNKSDFACANAFNNSIIPEDTFFPSPESSCDVKLVEK
SFETDTNLNFQNLSVIGFRILLLKVAGFNLLMTLRLWSS (SEQ ID NO:
61)
TCR7 α chain V region amino acid sequence 
DQQVKQNSPSLSVQEGRISILNCDYTNSMFDYFLWYKKYPAEGPT
FLISISSIKDKNEDGRFTVFLNKSAKHLSLHIVPSQPGDSAVYFCAGKTN
TGNQFYFGTGTSLTVIP (SEQ ID NO: 62)
TCR7 α chain CDR1 amino acid sequence
   NSMFDY (SEQ ID NO: 63)
TCR7 α chain CDR2 amino acid sequence
   ISSIKDK (SEQ ID NO: 64)
TCR7 α chain CDR3 amino acid sequence
   AGKTNTGNQFY (SEQ ID NO: 65)
TCR7 β chain amino acid sequence 
MGSRLLCWVLLCLLGAGPVKAGVTQTPRYLIKTRGQQVTLSCSPI
SGHRSVSWYQQTPGQGLQFLFEYFSETQRNKGNFPGRFSGRQFSNSRSE
MNVSTLELGDSALYLCASSSGHPEAFFGQGTRLTVVEDLNKVFPPEVA
VFEPSEAEISHTQKATLVCLATGFFPDHVELSWWVNGKEVHSGVSTDP
QPLKEQPALNDSRYCLSSRLRVSATFWQNPRNHFRCQVQFYGLSENDE
WTQDRAKPVTQIVSAEAWGRADCGFTSVSYQQGVLSATILYEILLGKA
TLYAVLVSALVLMAMVKRKDF (SEQ ID NO: 66)
TCR7 B chain V region amino acid sequence 
KAGVTQTPRYLIKTRGQQVTLSCSPISGHRSVSWYQQTPGQGLQFL
FEYFSETQRNKGNFPGRFSGRQFSNSRSEMNVSTLELGDSALYLCASSS
GHPEAFFGQGTRLTVV (SEQ ID NO: 67)
TCR7 β chain CDR1 amino acid sequence
   SGHRS (SEQ ID NO: 68)
TCR7 β chain CDR2 amino acid sequence
   YFSETQ (SEQ ID NO: 69)
TCR7 β chain CDR3 amino acid sequence
   ASSSGHPEAF (SEQ ID NO: 70)
TCR8 α chain amino acid sequence 
METLLGVSLVILWLQLARVNSQQGEEDPQALSIQEGENATMNCSY
KTSINNLQWYRQNSGRGLVHLILIRSNEREKHSGRLRVTLDTSKKSSSL
LITASRAADTASYFCATDAGGGADGLTFGKGTHLIIQPYIQNPDPAVYQ
LRDSKSSDKSVCLFTDFDSQTNVSQSKDSDVYITDKTVLDMRSMDFKS
NSAVAWSNKSDFACANAFNNSIIPEDTFFPSPESSCDVKLVEKSFETDTN
LNFQNLSVIGFRILLLKVAGFNLLMTLRLWSS (SEQ ID NO: 71)
TCR8 α chain V region amino acid sequence 
SQQGEEDPQALSIQEGENATMNCSYKTSINNLQWYRQNSGRGLVH
LILIRSNEREKHSGRLRVTLDTSKKSSSLLITASRAADTASYFCATDAGG
GADGLTFGKGTHLIIQP (SEQ ID NO: 72)
TCR8 α chain CDR1 amino acid sequence
   TSINN (SEQ ID NO: 73)
TCR8 α chain CDR2 amino acid sequence
   IRSNERE (SEQ ID NO: 74)
TCR8 α chain CDR3 amino acid sequence
   ATDAGGGADGLT (SEQ ID NO: 75)
TCR8 β chain amino acid sequence 
MGFRLLCCVAFCLLGAGPVDSGVTQTPKHLITATGQRVTLRCSPR
SGDLSVYWYQQSLDQGLQFLIQYYNGEERAKGNILERFSAQQFPDLHS
ELNLSSLELGDSALYFCASSESRDSGNTIYFGEGSWLTVVEDLNKVFPP
EVAVFEPSEAEISHTQKATLVCLATGFFPDHVELSWWVNGKEVHSGVS
TDPQPLKEQPALNDSRYCLSSRLRVSATFWQNPRNHFRCQVQFYGLSE
NDEWTQDRAKPVTQIVSAEAWGRADCGFTSVSYQQGVLSATILYEILL
GKATLYAVLVSALVLMAMVKRKDF (SEQ ID NO: 76)
TCR8 β chain V region amino acid sequence 
DSGVTQTPKHLITATGQRVTLRCSPRSGDLSVYWYQQSLDQGLQF
LIQYYNGEERAKGNILERFSAQQFPDLHSELNLSSLELGDSALYFCASSE
SRDSGNTIYFGEGSWLTVV (SEQ ID NO: 77)
TCR8 β chain CDR1 amino acid sequence
   SGDLS (SEQ ID NO: 78)
TCR8 β chain CDR2 amino acid sequence
   YYNGEE (SEQ ID NO: 79)
TCR8 β chain CDR3 amino acid sequence
   ASSESRDSGNTIY (SEQ ID NO: 80)
TCR9 α chain amino acid sequence 
METLLGVSLVILWLQLARVNSQQGEEDPQALSIQEGENATMNCSY
KTSINNLQWYRQNSGRGLVHLILIRSNEREKHSGRLRVTLDTSKKSSSL
LITASRAADTASYFCATDSGGGADGLTFGKGTHLIIQPYIQNPDPAVYQ
LRDSKSSDKSVCLFTDFDSQTNVSQSKDSDVYITDKTVLDMRSMDFKS
NSAVAWSNKSDFACANAFNNSIIPEDTFFPSPESSCDVKLVEKSFETDTN
LNFQNLSVIGFRILLLKVAGFNLLMTLRLWSS (SEQ ID NO: 81)
TCR9 α chain V region amino acid sequence 
SQQGEEDPQALSIQEGENATMNCSYKTSINNLQWYRQNSGRGLVH
LILIRSNEREKHSGRLRVTLDTSKKSSSLLITASRAADTASYFCATDSGG
GADGLTFGKGTHLIIQP (SEQ ID NO: 82)
TCR9 α chain CDR1 amino acid sequence
   TSINN (SEQ ID NO: 83)
TCR9 α chain CDR2 amino acid sequence
   IRSNERE (SEQ ID NO: 84)
TCR9 α chain CDR3 amino acid sequence
   ATDSGGGADGLT (SEQ ID NO: 85)
TCR9 β chain amino acid sequence 
MGFRLLCCVAFCLLGAGPVDSGVTQTPKHLITATGQRVTLRCSPR
SGDLSVYWYQQSLDQGLQFLIQYYNGEERAKGNILERFSAQQFPDLHS
ELNLSSLELGDSALYFCASTPGRDSGNTIYFGEGSWLTVVEDLNKVFPP
EVAVFEPSEAEISHTQKATLVCLATGFFPDHVELSWWVNGKEVHSGVS
TDPQPLKEQPALNDSRYCLSSRLRVSATFWQNPRNHFRCQVQFYGLSE
NDEWTQDRAKPVTQIVSAEAWGRADCGFTSVSYQQGVLSATILYEILL
GKATLYAVLVSALVLMAMVKRKDF (SEQ ID NO: 86)
TCR9 β chain V region amino acid sequence 
DSGVTQTPKHLITATGQRVTLRCSPRSGDLSVYWYQQSLDQGLQF
LIQYYNGEERAKGNILERFSAQQFPDLHSELNLSSLELGDSALYFCASTP
GRDSGNTIYFGEGSWLTVV (SEQ ID NO: 87)
TCR9 β chain CDR1 amino acid sequence
   SGDLS (SEQ ID NO: 88)
TCR9 β chain CDR2 amino acid sequence
   YYNGEE (SEQ ID NO: 89)
TCR9 β chain CDR3 amino acid sequence
   ASTPGRDSGNTIY (SEQ ID NO: 90)

## Claims

1. A binding protein, comprising a binding domain having antigen specificity for an antigen peptide:HLA complex, wherein the binding domain comprises a T cell receptor (TCR) α chain variable region and a TCR β chain variable region, wherein:
the antigen peptide comprises an amino acid sequence of VVGAVGVGK (SEQ ID NO: 91);
the HLA is HLA-A*11:01;
CDR3 of the α chain variable region comprises the amino acid sequence of SEQ ID NO: 5, 15, 25, 35, 45, 55, 65, 75, or 85, or an amino acid sequence having at least 80% identity, preferably at least 85% identity, more preferably 90% or 95% identity to the amino acid sequence of SEQ ID NO: 5, 15, 25, 35, 45, 55, 65, 75, or 85;
CDR3 of the β chain variable region comprises the amino acid sequence of SEQ ID NO: 10, 20, 30, 40, 50, 60, 70, 80, or 90, or an amino acid sequence having at least 80% identity, preferably at least 85% identity, more preferably 90% or 95% identity to the amino acid sequence of SEQ ID NO: 10, 20, 30, 40, 50, 60, 70, 80, or 90.

2. The binding protein of claim 1, wherein the antigen peptide is VVGAVGVGK (SEQ ID NO: 91) or VVVGAVGVGK (SEQ ID NO: 92).

3. The binding protein of claim 1, wherein the α chain variable region comprises:
1) CDR1 comprising the amino acid sequence of SEQ ID NO: 3, CDR2 comprising the amino acid sequence of SEQ ID NO: 4, and CDR3 comprising the amino acid sequence of SEQ ID NO: 5, or an amino acid sequence having at least 80% identity, preferably at least 85% identity, more preferably 90% or 95% identity to the amino acid sequence of SEQ ID NO: 5;
2) CDR1 comprising the amino acid sequence of SEQ ID NO: 13, CDR2 comprising the amino acid sequence of SEQ ID NO: 14, and CDR3 comprising the amino acid sequence of SEQ ID NO: 15, or an amino acid sequence having at least 80% identity, preferably at least 85% identity, more preferably 90% or 95% identity to the amino acid sequence of SEQ ID NO: 15;
3) CDR1 comprising the amino acid sequence of SEQ ID NO: 23, CDR2 comprising the amino acid sequence of SEQ ID NO: 24, and CDR3 comprising the amino acid sequence of SEQ ID NO: 25, or an amino acid sequence having at least 80% identity, preferably at least 85% identity, more preferably 90% or 95% identity to the amino acid sequence of SEQ ID NO: 25;
4) CDR1 comprising the amino acid sequence of SEQ ID NO: 33, CDR2 comprising the amino acid sequence of SEQ ID NO: 34, and CDR3 comprising the amino acid sequence of SEQ ID NO: 35, or an amino acid sequence having at least 80% identity, preferably at least 85% identity, more preferably 90% or 95% identity to the amino acid sequence of SEQ ID NO: 35;
5) CDR1 comprising the amino acid sequence of SEQ ID NO: 43, CDR2 comprising the amino acid sequence of SEQ ID NO: 44, and CDR3 comprising the amino acid sequence of SEQ ID NO: 45, or an amino acid sequence having at least 80% identity, preferably at least 85% identity, more preferably 90% or 95% identity to the amino acid sequence of SEQ ID NO: 45;
6) CDR1 comprising the amino acid sequence of SEQ ID NO: 53, CDR2 comprising the amino acid sequence of SEQ ID NO: 54, and CDR3 comprising the amino acid sequence of SEQ ID NO: 55, or an amino acid sequence having at least 80% identity, preferably at least 85% identity, more preferably 90% or 95% identity to the amino acid sequence of SEQ ID NO: 55;
7) CDR1 comprising the amino acid sequence of SEQ ID NO: 63, CDR2 comprising the amino acid sequence of SEQ ID NO: 64, and CDR3 comprising the amino acid sequence of SEQ ID NO: 65, or an amino acid sequence having at least 80% identity, preferably at least 85% identity, more preferably 90% or 95% identity to the amino acid sequence of SEQ ID NO: 65;
8) CDR1 comprising the amino acid sequence of SEQ ID NO: 73, CDR2 comprising the amino acid sequence of SEQ ID NO: 74, and CDR3 comprising the amino acid sequence of SEQ ID NO: 75, or an amino acid sequence having at least 80% identity, preferably at least 85% identity, more preferably 90% or 95% identity to the amino acid sequence of SEQ ID NO: 75; or
9) CDR1 comprising the amino acid sequence of SEQ ID NO: 83, CDR2 comprising the amino acid sequence of SEQ ID NO: 84, and CDR3 comprising the amino acid sequence of SEQ ID NO: 85, or an amino acid sequence having at least 80% identity, preferably at least 85% identity, more preferably 90% or 95% identity to the amino acid sequence of SEQ ID NO: 85, and
the β chain variable region comprises:
1) CDR1 comprising the amino acid sequence of SEQ ID NO: 8, CDR2 comprising the amino acid sequence of SEQ ID NO: 9, and CDR3 comprising the amino acid sequence of SEQ ID NO: 10, or an amino acid sequence having at least 80% identity, preferably at least 85% identity, more preferably 90% or 95% identity to the amino acid sequence of SEQ ID NO: 10;
2) CDR1 comprising the amino acid sequence of SEQ ID NO: 18, CDR2 comprising the amino acid sequence of SEQ ID NO: 19, and CDR3 comprising the amino acid sequence of SEQ ID NO: 20, or an amino acid sequence having at least 80% identity, preferably at least 85% identity, more preferably 90% or 95% identity to the amino acid sequence of SEQ ID NO: 20;
3) CDR1 comprising the amino acid sequence of SEQ ID NO: 28, CDR2 comprising the amino acid sequence of SEQ ID NO: 29, and CDR3 comprising the amino acid sequence of SEQ ID NO: 30, or an amino acid sequence having at least 80% identity, preferably at least 85% identity, more preferably 90% or 95% identity to the amino acid sequence of SEQ ID NO: 30;
4) CDR1 comprising the amino acid sequence of SEQ ID NO: 38, CDR2 comprising the amino acid sequence of SEQ ID NO: 39, and CDR3 comprising the amino acid sequence of SEQ ID NO: 40, or an amino acid sequence having at least 80% identity, preferably at least 85% identity, more preferably 90% or 95% identity to the amino acid sequence of SEQ ID NO: 40;
5) CDR1 comprising the amino acid sequence of SEQ ID NO: 48, CDR2 comprising the amino acid sequence of SEQ ID NO: 49, and CDR3 comprising the amino acid sequence of SEQ ID NO: 50, or an amino acid sequence having at least 80% identity, preferably at least 85% identity, more preferably 90% or 95% identity to the amino acid sequence of SEQ ID NO: 50;
6) CDR1 comprising the amino acid sequence of SEQ ID NO: 58, CDR2 comprising the amino acid sequence of SEQ ID NO: 59, and CDR3 comprising the amino acid sequence of SEQ ID NO: 60, or an amino acid sequence having at least 80% identity, preferably at least 85% identity, more preferably 90% or 95% identity to the amino acid sequence of SEQ ID NO: 60;
7) CDR1 comprising the amino acid sequence of SEQ ID NO: 68, CDR2 comprising the amino acid sequence of SEQ ID NO: 69, and CDR3 comprising the amino acid sequence of SEQ ID NO: 70, or an amino acid sequence having at least 80% identity, preferably at least 85% identity, more preferably 90% or 95% identity to the amino acid sequence of SEQ ID NO: 70;
8) CDR1 comprising the amino acid sequence of SEQ ID NO: 78, CDR2 comprising the amino acid sequence of SEQ ID NO: 79, and CDR3 comprising the amino acid sequence of SEQ ID NO: 80, or an amino acid sequence having at least 80% identity, preferably at least 85% identity, more preferably 90% or 95% identity to the amino acid sequence of SEQ ID NO: 80; or
9) CDR1 comprising the amino acid sequence of SEQ ID NO: 88, CDR2 comprising the amino acid sequence of SEQ ID NO: 89, and CDR3 comprising the amino acid sequence of SEQ ID NO: 90, or an amino acid sequence having at least 80% identity, preferably at least 85% identity, more preferably 90% or 95% identity to the amino acid sequence of SEQ ID NO: 90.

4. The binding protein of any one of claims 1-3, wherein:
1) the α chain variable region comprises CDR1 comprising the amino acid sequence of SEQ ID NO: 3, CDR2 comprising the amino acid sequence of SEQ ID NO: 4, and CDR3 comprising the amino acid sequence of SEQ ID NO: 5, or an amino acid sequence having at least 80% identity, preferably at least 85% identity, more preferably 90% or 95% identity to the amino acid sequence of SEQ ID NO: 5; the β chain variable region comprises CDR1 comprising the amino acid sequence of SEQ ID NO: 8, CDR2 comprising the amino acid sequence of SEQ ID NO: 9, and CDR3 comprising the amino acid sequence of SEQ ID NO: 10, or an amino acid sequence having at least 80% identity, preferably at least 85% identity, more preferably 90% or 95% identity to the amino acid sequence of SEQ ID NO: 10;
2) the α chain variable region comprises CDR1 comprising the amino acid sequence of SEQ ID NO: 13, CDR2 comprising the amino acid sequence of SEQ ID NO: 14, and CDR3 comprising the amino acid sequence of SEQ ID NO: 15, or an amino acid sequence having at least 80% identity, preferably at least 85% identity, more preferably 90% or 95% identity to the amino acid sequence of SEQ ID NO: 15; the β chain variable region comprises CDR1 comprising the amino acid sequence of SEQ ID NO: 18, CDR2 comprising the amino acid sequence of SEQ ID NO: 19, and CDR3 comprising the amino acid sequence of SEQ ID NO: 20, or an amino acid sequence having at least 80% identity, preferably at least 85% identity, more preferably 90% or 95% identity to the amino acid sequence of SEQ ID NO: 20;
3) the α chain variable region comprises CDR1 comprising the amino acid sequence of SEQ ID NO: 23, CDR2 comprising the amino acid sequence of SEQ ID NO: 24, and CDR3 comprising the amino acid sequence of SEQ ID NO: 25, or an amino acid sequence having at least 80% identity, preferably at least 85% identity, more preferably 90% or 95% identity to the amino acid sequence of SEQ ID NO: 25; the β chain variable region comprises CDR1 comprising the amino acid sequence of SEQ ID NO: 28, CDR2 comprising the amino acid sequence of SEQ ID NO: 29, and CDR3 comprising the amino acid sequence of SEQ ID NO: 30, or an amino acid sequence having at least 80% identity, preferably at least 85% identity, more preferably 90% or 95% identity to the amino acid sequence of SEQ ID NO: 30;
4) the α chain variable region comprises CDR1 comprising the amino acid sequence of SEQ ID NO: 33, CDR2 comprising the amino acid sequence of SEQ ID NO: 34, and CDR3 comprising the amino acid sequence of SEQ ID NO: 35, or an amino acid sequence having at least 80% identity, preferably at least 85% identity, more preferably 90% or 95% identity to the amino acid sequence of SEQ ID NO: 35; the β chain variable region comprises CDR1 comprising the amino acid sequence of SEQ ID NO: 38, CDR2 comprising the amino acid sequence of SEQ ID NO: 39, and CDR3 comprising the amino acid sequence of SEQ ID NO: 40, or an amino acid sequence having at least 80% identity, preferably at least 85% identity, more preferably 90% or 95% identity to the amino acid sequence of SEQ ID NO: 40;
5) the α chain variable region comprises CDR1 comprising the amino acid sequence of SEQ ID NO: 43, CDR2 comprising the amino acid sequence of SEQ ID NO: 44, and CDR3 comprising the amino acid sequence of SEQ ID NO: 45, or an amino acid sequence having at least 80% identity, preferably at least 85% identity, more preferably 90% or 95% identity to the amino acid sequence of SEQ ID NO: 45; the β chain variable region comprises CDR1 comprising the amino acid sequence of SEQ ID NO: 48, CDR2 comprising the amino acid sequence of SEQ ID NO: 49, and CDR3 comprising the amino acid sequence of SEQ ID NO: 50, or an amino acid sequence having at least 80% identity, preferably at least 85% identity, more preferably 90% or 95% identity to the amino acid sequence of SEQ ID NO: 50;
6) the α chain variable region comprises CDR1 comprising the amino acid sequence of SEQ ID NO: 53, CDR2 comprising the amino acid sequence of SEQ ID NO: 54, and CDR3 comprising the amino acid sequence of SEQ ID NO: 55, or an amino acid sequence having at least 80% identity, preferably at least 85% identity, more preferably 90% or 95% identity to the amino acid sequence of SEQ ID NO: 55; the β chain variable region comprises CDR1 comprising the amino acid sequence of SEQ ID NO: 58, CDR2 comprising the amino acid sequence of SEQ ID NO: 59, and CDR3 comprising the amino acid sequence of SEQ ID NO: 60, or an amino acid sequence having at least 80% identity, preferably at least 85% identity, more preferably 90% or 95% identity to the amino acid sequence of SEQ ID NO: 60;
7) the α chain variable region comprises CDR1 comprising the amino acid sequence of SEQ ID NO: 63, CDR2 comprising the amino acid sequence of SEQ ID NO: 64, and CDR3 comprising the amino acid sequence of SEQ ID NO: 65, or an amino acid sequence having at least 80% identity, preferably at least 85% identity, more preferably 90% or 95% identity to the amino acid sequence of SEQ ID NO: 65; the β chain variable region comprises CDR1 comprising the amino acid sequence of SEQ ID NO: 68, CDR2 comprising the amino acid sequence of SEQ ID NO: 69, and CDR3 comprising the amino acid sequence of SEQ ID NO: 70, or an amino acid sequence having at least 80% identity, preferably at least 85% identity, more preferably 90% or 95% identity to the amino acid sequence of SEQ ID NO: 70;
8) the α chain variable region comprises CDR1 comprising the amino acid sequence of SEQ ID NO: 73, CDR2 comprising the amino acid sequence of SEQ ID NO: 74, and CDR3 comprising the amino acid sequence of SEQ ID NO: 75, or an amino acid sequence having at least 80% identity, preferably at least 85% identity, more preferably 90% or 95% identity to the amino acid sequence of SEQ ID NO: 75; the β chain variable region comprises CDR1 comprising the amino acid sequence of SEQ ID NO: 78, CDR2 comprising the amino acid sequence of SEQ ID NO: 79, and CDR3 comprising the amino acid sequence of SEQ ID NO: 80, or an amino acid sequence having at least 80% identity, preferably at least 85% identity, more preferably 90% or 95% identity to the amino acid sequence of SEQ ID NO: 80; or
9) the α chain variable region comprises CDR1 comprising the amino acid sequence of SEQ ID NO: 83, CDR2 comprising the amino acid sequence of SEQ ID NO: 84, and CDR3 comprising the amino acid sequence of SEQ ID NO: 85, or an amino acid sequence having at least 80% identity, preferably at least 85% identity, more preferably 90% or 95% identity to the amino acid sequence of SEQ ID NO: 85; the β chain variable region comprises CDR1 comprising the amino acid sequence of SEQ ID NO: 88, CDR2 comprising the amino acid sequence of SEQ ID NO: 89, and CDR3 comprising the amino acid sequence of SEQ ID NO: 90, or an amino acid sequence having at least 80% identity, preferably at least 85% identity, more preferably 90% or 95% identity to the amino acid sequence of SEQ ID NO: 90.

5. A binding protein comprising a binding domain, wherein the binding domain comprises a T cell receptor (TCR) α chain variable region and a TCR β-chain variable region, wherein:
1) the α chain variable region comprises CDR1 comprising the amino acid sequence of SEQ ID NO: 3, CDR2 comprising the amino acid sequence of SEQ ID NO: 4, and CDR3 comprising the amino acid sequence of SEQ ID NO: 5, or an amino acid sequence having at least 80% identity, preferably at least 85% identity, more preferably 90% or 95% identity to the amino acid sequence of SEQ ID NO: 5; the β chain variable region comprises CDR1 comprising the amino acid sequence of SEQ ID NO: 8, CDR2 comprising the amino acid sequence of SEQ ID NO: 9, and CDR3 comprising the amino acid sequence of SEQ ID NO: 10, or an amino acid sequence having at least 80% identity, preferably at least 85% identity, more preferably 90% or 95% identity to the amino acid sequence of SEQ ID NO: 10;
2) the α chain variable region comprises CDR1 comprising the amino acid sequence of SEQ ID NO: 13, CDR2 comprising the amino acid sequence of SEQ ID NO: 14, and CDR3 comprising the amino acid sequence of SEQ ID NO: 15, or an amino acid sequence having at least 80% identity, preferably at least 85% identity, more preferably 90% or 95% identity to the amino acid sequence of SEQ ID NO: 15; the β chain variable region comprises CDR1 comprising the amino acid sequence of SEQ ID NO: 18, CDR2 comprising the amino acid sequence of SEQ ID NO: 19, and CDR3 comprising the amino acid sequence of SEQ ID NO: 20, or an amino acid sequence having at least 80% identity, preferably at least 85% identity, more preferably 90% or 95% identity to the amino acid sequence of SEQ ID NO: 20;
3) the α chain variable region comprises CDR1 comprising the amino acid sequence of SEQ ID NO: 23, CDR2 comprising the amino acid sequence of SEQ ID NO: 24, and CDR3 comprising the amino acid sequence of SEQ ID NO: 25, or an amino acid sequence having at least 80% identity, preferably at least 85% identity, more preferably 90% or 95% identity to the amino acid sequence of SEQ ID NO: 25; the β chain variable region comprises CDR1 comprising the amino acid sequence of SEQ ID NO: 28, CDR2 comprising the amino acid sequence of SEQ ID NO: 29, and CDR3 comprising the amino acid sequence of SEQ ID NO: 30, or an amino acid sequence having at least 80% identity, preferably at least 85% identity, more preferably 90% or 95% identity to the amino acid sequence of SEQ ID NO: 30;
4) the α chain variable region comprises CDR1 comprising the amino acid sequence of SEQ ID NO: 33, CDR2 comprising the amino acid sequence of SEQ ID NO: 34, and CDR3 comprising the amino acid sequence of SEQ ID NO: 35, or an amino acid sequence having at least 80% identity, preferably at least 85% identity, more preferably 90% or 95% identity to the amino acid sequence of SEQ ID NO: 35; the β chain variable region comprises CDR1 comprising the amino acid sequence of SEQ ID NO: 38, CDR2 comprising the amino acid sequence of SEQ ID NO: 39, and CDR3 comprising the amino acid sequence of SEQ ID NO: 40, or an amino acid sequence having at least 80% identity, preferably at least 85% identity, more preferably 90% or 95% identity to the amino acid sequence of SEQ ID NO: 40;
5) the α chain variable region comprises CDR1 comprising the amino acid sequence of SEQ ID NO: 43, CDR2 comprising the amino acid sequence of SEQ ID NO: 44, and CDR3 comprising the amino acid sequence of SEQ ID NO: 45, or an amino acid sequence having at least 80% identity, preferably at least 85% identity, more preferably 90% or 95% identity to the amino acid sequence of SEQ ID NO: 45; the β chain variable region comprises CDR1 comprising the amino acid sequence of SEQ ID NO: 48, CDR2 comprising the amino acid sequence of SEQ ID NO: 49, and CDR3 comprising the amino acid sequence of SEQ ID NO: 50, or an amino acid sequence having at least 80% identity, preferably at least 85% identity, more preferably 90% or 95% identity to the amino acid sequence of SEQ ID NO: 50;
6) the α chain variable region comprises CDR1 comprising the amino acid sequence of SEQ ID NO: 53, CDR2 comprising the amino acid sequence of SEQ ID NO: 54, and CDR3 comprising the amino acid sequence of SEQ ID NO: 55, or an amino acid sequence having at least 80% identity, preferably at least 85% identity, more preferably 90% or 95% identity to the amino acid sequence of SEQ ID NO: 55; the β chain variable region comprises CDR1 comprising the amino acid sequence of SEQ ID NO: 58, CDR2 comprising the amino acid sequence of SEQ ID NO: 59, and CDR3 comprising the amino acid sequence of SEQ ID NO: 60, or an amino acid sequence having at least 80% identity, preferably at least 85% identity, more preferably 90% or 95% identity to the amino acid sequence of SEQ ID NO: 60;
7) the α chain variable region comprises CDR1 comprising the amino acid sequence of SEQ ID NO: 63, CDR2 comprising the amino acid sequence of SEQ ID NO: 64, and CDR3 comprising the amino acid sequence of SEQ ID NO: 65, or an amino acid sequence having at least 80% identity, preferably at least 85% identity, more preferably 90% or 95% identity to the amino acid sequence of SEQ ID NO: 65; the β chain variable region comprises CDR1 comprising the amino acid sequence of SEQ ID NO: 68, CDR2 comprising the amino acid sequence of SEQ ID NO: 69, and CDR3 comprising the amino acid sequence of SEQ ID NO: 70, or an amino acid sequence having at least 80% identity, preferably at least 85% identity, more preferably 90% or 95% identity to the amino acid sequence of SEQ ID NO: 70;
8) the α chain variable region comprises CDR1 comprising the amino acid sequence of SEQ ID NO: 73, CDR2 comprising the amino acid sequence of SEQ ID NO: 74, and CDR3 comprising the amino acid sequence of SEQ ID NO: 75, or an amino acid sequence having at least 80% identity, preferably at least 85% identity, more preferably 90% or 95% identity to the amino acid sequence of SEQ ID NO: 75; the β chain variable region comprises CDR1 comprising the amino acid sequence of SEQ ID NO: 78, CDR2 comprising the amino acid sequence of SEQ ID NO: 79, and CDR3 comprising the amino acid sequence of SEQ ID NO: 80, or an amino acid sequence having at least 80% identity, preferably at least 85% identity, more preferably 90% or 95% identity to the amino acid sequence of SEQ ID NO: 80; or
9) the α chain variable region comprises CDR1 comprising the amino acid sequence of SEQ ID NO: 83, CDR2 comprising the amino acid sequence of SEQ ID NO: 84, and CDR3 comprising the amino acid sequence of SEQ ID NO: 85, or an amino acid sequence having at least 80% identity, preferably at least 85% identity, more preferably 90% or 95% identity to the amino acid sequence of SEQ ID NO: 85; the β chain variable region comprises CDR1 comprising the amino acid sequence of SEQ ID NO: 88, CDR2 comprising the amino acid sequence of SEQ ID NO: 89, and CDR3 comprising the amino acid sequence of SEQ ID NO: 90, or an amino acid sequence having at least 80% identity, preferably at least 85% identity, more preferably 90% or 95% identity to the amino acid sequence of SEQ ID NO: 90.

6. The binding protein of any one of claims 1-5, wherein:
1) the α chain variable region comprises the amino acid sequence of SEQ ID NO: 2 or an amino acid sequence having at least 80% identity, preferably at least 85% identity, more preferably 90% or 95% identity to the amino acid sequence of SEQ ID NO: 2; the β chain variable region comprises the amino acid sequence of SEQ ID NO: 7 or an amino acid sequence having at least 80% identity, preferably at least 85% identity, more preferably 90% or 95% identity to the amino acid sequence of SEQ ID NO: 7;
2) the α chain variable region comprises the amino acid sequence of SEQ ID NO: 12 or an amino acid sequence having at least 80% identity, preferably at least 85% identity, more preferably 90% or 95% identity to the amino acid sequence of SEQ ID NO: 12; the β chain variable region comprises the amino acid sequence of SEQ ID NO: 17 or an amino acid sequence having at least 80% identity, preferably at least 85% identity, more preferably 90% or 95% identity to the amino acid sequence of SEQ ID NO: 17;
3) the α chain variable region comprises the amino acid sequence of SEQ ID NO: 22 or an amino acid sequence having at least 80% identity, preferably at least 85% identity, more preferably 90% or 95% identity to the amino acid sequence of SEQ ID NO: 22; the β chain variable region comprises the amino acid sequence of SEQ ID NO: 27 or an amino acid sequence having at least 80% identity, preferably at least 85% identity, more preferably 90% or 95% identity to the amino acid sequence of SEQ ID NO: 27;
4) the α chain variable region comprises the amino acid sequence of SEQ ID NO: 32 or an amino acid sequence having at least 80% identity, preferably at least 85% identity, more preferably 90% or 95% identity to the amino acid sequence of SEQ ID NO: 32; the β chain variable region comprises the amino acid sequence of SEQ ID NO: 37 or an amino acid sequence having at least 80% identity, preferably at least 85% identity, more preferably 90% or 95% identity to the amino acid sequence of SEQ ID NO: 37;
5) the α chain variable region comprises the amino acid sequence of SEQ ID NO: 42 or an amino acid sequence having at least 80% identity, preferably at least 85% identity, more preferably 90% or 95% identity to the amino acid sequence of SEQ ID NO: 42; the β chain variable region comprises the amino acid sequence of SEQ ID NO: 47 or an amino acid sequence having at least 80% identity, preferably at least 85% identity, more preferably 90% or 95% identity to the amino acid sequence of SEQ ID NO: 47;
6) the α chain variable region comprises the amino acid sequence of SEQ ID NO: 52 or an amino acid sequence having at least 80% identity, preferably at least 85% identity, more preferably 90% or 95% identity to the amino acid sequence of SEQ ID NO: 52; the β chain variable region comprises the amino acid sequence of SEQ ID NO: 57 or an amino acid sequence having at least 80% identity, preferably at least 85% identity, more preferably 90% or 95% identity to the amino acid sequence of SEQ ID NO: 57;
7) the α chain variable region comprises the amino acid sequence of SEQ ID NO: 62 or an amino acid sequence having at least 80% identity, preferably at least 85% identity, more preferably 90% or 95% identity to the amino acid sequence of SEQ ID NO: 62; the β chain variable region comprises the amino acid sequence of SEQ ID NO: 67 or an amino acid sequence having at least 80% identity, preferably at least 85% identity, more preferably 90% or 95% identity to the amino acid sequence of SEQ ID NO: 67;
8) the α chain variable region comprises the amino acid sequence of SEQ ID NO: 72 or an amino acid sequence having at least 80% identity, preferably at least 85% identity, more preferably 90% or 95% identity to the amino acid sequence of SEQ ID NO: 72; the β chain variable region comprises the amino acid sequence of SEQ ID NO: 77 or an amino acid sequence having at least 80% identity, preferably at least 85% identity, more preferably 90% or 95% identity to the amino acid sequence of SEQ ID NO: 77; or
9) the α chain variable region comprises the amino acid sequence of SEQ ID NO: 82 or an amino acid sequence having at least 80% identity, preferably at least 85% identity, more preferably 90% or 95% identity to the amino acid sequence of SEQ ID NO: 82; the β chain variable region comprises the amino acid sequence of SEQ ID NO: 87 or an amino acid sequence having at least 80% identity, preferably at least 85% identity, more preferably 90% or 95% identity to the amino acid sequence of SEQ ID NO: 87.

7. The binding protein of any one of claims 1-6, comprising the TCR α chain and TCR β chain, wherein:
1) the α chain comprises the amino acid sequence of SEQ ID NO: 1 or an amino acid sequence having at least 80% identity, preferably at least 85% identity, more preferably 90% or 95% identity to the amino acid sequence of SEQ ID NO: 1; the β chain comprises the amino acid sequence of SEQ ID NO: 6 or an amino acid sequence having at least 80% identity, preferably at least 85% identity, more preferably 90% or 95% identity to the amino acid sequence of SEQ ID NO: 6;
2) the α chain comprises the amino acid sequence of SEQ ID NO: 11 or an amino acid sequence having at least 80% identity, preferably at least 85% identity, more preferably 90% or 95% identity to the amino acid sequence of SEQ ID NO: 11; the β chain comprises the amino acid sequence of SEQ ID NO: 16 or an amino acid sequence having at least 80% identity, preferably at least 85% identity, more preferably 90% or 95% identity to the amino acid sequence of SEQ ID NO: 16;
3) the α chain comprises the amino acid sequence of SEQ ID NO: 21 or an amino acid sequence having at least 80% identity, preferably at least 85% identity, more preferably 90% or 95% identity to the amino acid sequence of SEQ ID NO: 21; the β chain comprises the amino acid sequence of SEQ ID NO: 26 or an amino acid sequence having at least 80% identity, preferably at least 85% identity, more preferably 90% or 95% identity to the amino acid sequence of SEQ ID NO: 26;
4) the α chain comprises the amino acid sequence of SEQ ID NO: 31 or an amino acid sequence having at least 80% identity, preferably at least 85% identity, more preferably 90% or 95% identity to the amino acid sequence of SEQ ID NO: 31; the β chain comprises the amino acid sequence of SEQ ID NO: 36 or an amino acid sequence having at least 80% identity, preferably at least 85% identity, more preferably 90% or 95% identity to the amino acid sequence of SEQ ID NO: 36;
5) the α chain comprises the amino acid sequence of SEQ ID NO: 41 or an amino acid sequence having at least 80% identity, preferably at least 85% identity, more preferably 90% or 95% identity to the amino acid sequence of SEQ ID NO: 41; the β chain comprises the amino acid sequence of SEQ ID NO: 46 or an amino acid sequence having at least 80% identity, preferably at least 85% identity, more preferably 90% or 95% identity to the amino acid sequence of SEQ ID NO: 46;
6) the α chain comprises the amino acid sequence of SEQ ID NO: 51 or an amino acid sequence having at least 80% identity, preferably at least 85% identity, more preferably 90% or 95% identity to the amino acid sequence of SEQ ID NO: 51; the β chain comprises the amino acid sequence of SEQ ID NO: 56 or an amino acid sequence having at least 80% identity, preferably at least 85% identity, more preferably 90% or 95% identity to the amino acid sequence of SEQ ID NO: 56;
7) the α chain comprises the amino acid sequence of SEQ ID NO: 61 or an amino acid sequence having at least 80% identity, preferably at least 85% identity, more preferably 90% or 95% identity to the amino acid sequence of SEQ ID NO: 61; the β chain comprises the amino acid sequence of SEQ ID NO: 66 or an amino acid sequence having at least 80% identity, preferably at least 85% identity, more preferably 90% or 95% identity to the amino acid sequence of SEQ ID NO: 66;
8) the α chain comprises the amino acid sequence of SEQ ID NO: 71 or an amino acid sequence having at least 80% identity, preferably at least 85% identity, more preferably 90% or 95% identity to the amino acid sequence of SEQ ID NO: 71; the β chain comprises the amino acid sequence of SEQ ID NO: 76 or an amino acid sequence having at least 80% identity, preferably at least 85% identity, more preferably 90% or 95% identity to the amino acid sequence of SEQ ID NO: 76; or
9) the α chain comprises the amino acid sequence of SEQ ID NO: 81 or an amino acid sequence having at least 80% identity, preferably at least 85% identity, more preferably 90% or 95% identity to the amino acid sequence of SEQ ID NO: 81; the β chain comprises the amino acid sequence of SEQ ID NO: 86 or an amino acid sequence having at least 80% identity, preferably at least 85% identity, more preferably 90% or 95% identity to the amino acid sequence of SEQ ID NO: 86.

8. The binding protein of any one of claims 1-7, wherein the binding protein is a protein in the form of:
1) a TCR molecule;
2) an antibody molecule; or
3) a CAR molecule.

9. The binding protein of any one of claims 1-8, wherein when a T cell expressing the binding protein is co-incubated with an HLA-A*11:01 cells in the presence of the antigen peptide, or when the T cell expressing the binding protein is co-incubated with the HLA-A*11:01 cell expressing the antigen peptide, the T cell specifically kills the HLA-A*11:01 cell.

10. The binding protein of any one of claims 1-9, wherein when a T cell expressing the binding protein is co-incubated with an HLA-A*11:01 cell in the presence of the antigen peptide, or when the T cell expressing the binding protein is co-incubated with the HLA-A*11:01 cell, the T cell produces IFN-γ and/or expresses CD69, or exhibits increased production of IFN-γ and/or expression of CD69.

11. The binding protein of any one of claims 1-10, which further comprises a conjugate covalently or non-covalently linked to the binding domain; preferably, the conjugate is a detectable label, a radioisotope, or a therapeutic agent.

12. An isolated nucleic acid molecule, which encodes the binding protein of any one of claims 1-11, the α chain variable region, the β chain variable region, the α chain, or the β chain.

13. A vector comprising the nucleic acid molecule of claim 12.

14. A host cell expressing the binding protein of any one of claims 1-11, the nucleic acid molecule of claim 12, or the vector of claim 13.

15. The host cell of claim 14, wherein the host cell is a mammalian cell, preferably a human cell.

16. The host cell of claim 14 or claim 15, wherein the host cell is a T cell or an NK cell.

17. The host cell of any one of claims 14-16, wherein the host cell exhibits cytotoxic activity against HLA-A*11:01 cells expressing the antigen peptide VVGAVGVGK (SEQ ID NO: 91) or VVVGAVGVGK (SEQ ID NO: 92).

18. A pharmaceutical composition comprising:
1) the binding protein of any one of claims 1-11, the nucleic acid molecule of claim 12, the vector of claim 13, or the host cell of any one of claims 14-17; and
2) a pharmaceutically acceptable carrier.

19. Use of the binding protein of any one of claims 1-11, the nucleic acid molecule of claim 12, the vector of claim 13, or the host cell of any one of claims 14-17 in the manufacture of a medicament for treating a tumor, wherein the tumor expresses the antigen peptide.

20. A method for treating a tumor in a subject, comprising administering to the subject a therapeutically effective amount of the binding protein of any one of claims 1-11, the nucleic acid molecule of claim 12, the vector of claim 13, the host cell of any one of claims 14-17, or the pharmaceutical composition of claim 18.

21. The use of claim 19 or the method of claim 20, wherein the tumor is selected from pancreatic cancer, colorectal cancer, lung cancer, cholangiocarcinoma, endometrial cancer, and ovarian cancer.

22. A detection kit comprising the binding protein of any one of claims 1-11.
